# EUROPEAN PATENT SPECIFICATION

(11) **EP 3 217 972 B1**
(45) Date of publication and mention of the grant of the patent: **24.06.2020**
(21) Application number: 15859697.3
(22) Date of filing: 12.11.2015
(51) Int. Cl.: A61K 31/365, A61K 31/366, C07C 51/09, C07C 31/20, C07C 31/22, C07C 33/035, C07C 235/08, C07C 235/10, C07D 303/16, C07D 303/28, C07C 55/02, C07C 57/13, C08G 63/16, C08G 63/52, C08G 69/44, C08G 63/60

(54) **POLYMERS PREPARED FROM MEVALONOLACTONE AND DERIVATIVES**
AUS MEVALONOLACTON HERGESTELLTE POLYMERE UND DERIVATE
POLYMÈRES PRÉPARÉS À PARTIR DE LA MÉVALONOLACTONE ET DE SES DÉRIVÉS

(30) Priority: 12.11.2014 US 201462078511 P
(43) Date of publication of application: 20.09.2017
(73) Proprietor: Visolis, Inc., Cambridge, Massachusetts 02139 (US)
(72) Inventor: DUGAR, Deepak, Berkeley, CA 94704 (US); FRIEDBERGER, Tobias, Oakland, California 94602 (US)
(74) Representative: Hocking, Adrian Niall
(86) International application number: PCT/US2015/060335
(87) International publication number: WO 2016/077555

(56) References cited:
- EP-A1- 1 471 052
- WO-A2-2011/100327
- CN-A- 1 781 927
- M. XIONG ET AL: "Scalable production of mechanically tunable block polymers from sugar", PROCEEDINGS OF THE NATIONAL ACADEMY OF SCIENCES, vol. 111, no. 23, 27 May 2014 (2014-05-27) , pages 8357-8362, XP055198940, ISSN: 0027-8424, DOI: 10.1073/pnas.1404596111
- C. Foote ET AL: "The Chemical Synthesis of Mevalonic Acid 5-Phosphate, Isopentenyl Pyrophosphate, and Related Compounds", Biochemistry, vol. 2, n. 6, 8 July 1963 (1963-07-08), pages 1254-1258, XP055441165, Retrieved from the Internet: URL:https://pubs.acs.org/doi/abs/10.1021/b i00906a014 [retrieved on 2018-01-16]
- FOOTE, CD ET AL.: 'The Chemical Synthesis of Mevalonic Acid 5-Phosphate, Isopentenyl Pyrophosphate, and Related Compounds' BIOCHEMISTRY vol. 2, no. 6, 08 July 1963, pages 1254 - 1258, XP055441165 Retrieved from the Internet: <URL:http://pubs.acs.org/doi/abs/10.1021/bi 00906a014> [retrieved on 2015-12-21]
- JEAN, M ET AL.: 'Synthesis of a Laterally Branched Polyamine from alpha-Methylene-gama-butyrolactone.' ORGANIC LETTERS vol. 7, no. 13, 2005, pages 2663 - 2665, XP055441172 Retrieved from the Internet: <URL:https://www.researchgate.net/profile/M ickael_Jean/publication/7784786_Synthesis_o f_a_laterall y_branched_polyamine_from_alpha-methylene-g amma-butyrolactone/links/0046351bae79216d b5000000.pdf> [retrieved on 2015-12-21]

## Description

### BACKGROUND OF THE INVENTION

Many existing chemical products, such as surfactants, plasticizers, solvents, and polymers, are currently manufactured from non-renewable, expensive, petroleum-derived or natural gas-derived feedstock materials. High raw material costs and uncertainty of future supplies requires the discovery and development of surfactants, plasticizers, solvents, polymers and thermosets that can be made from inexpensive and renewable compounds that are readily available.

Lactones are an important class of compounds that can be derived from biomass via biochemical routes and can serve as intermediates for the sustainable production of hydrocarbon biofuels and other products. In particular, mevalonolactone can be derived from biomass fermentation to produce a variety of intermediates. Mevalonolactone (MVL) and its related derivatives, such as 2,3-dehydromevalonolactone (4-methyl-5,6-dihydro-2H-pyran-2-one; also referred to as anhydromevalonolactone, AML) represent a potentially abundant feedstock that may be prepared on an industrial scale.

Mevalonic acid, mevalonate, and mevalonolactone exist in an equilibrium which is pH dependent. Thus, a solution which contains "mevalonate" may actually contain mevalonic acid, mevalonate, and/or mevalonolactone. For convenience, mevalonolactone or "MVL" will be used herein inclusively. In addition, depending on the pH, the components may be in salt form. Depending on the reagent chosen, the counter ion can be the cation of ammonium, sodium, lithium, potassium, magnesium, calcium, aluminum, or cesium. Biobased mevalonolactone can subsequently be converted into a variety of useful compounds. In Biochemistry, vol. 2, n.6, pg. 1254-1258 the chemical synthesis of mevalonic acid 5-phosphate and related compounds is disclosed. Exemplary processes are known in the literature, which a preferred process is described in US 2019169153.

### SUMMARY OF THE INVENTION

Biobased production via fermentation allows for the high-yielding and cost competitive production of MVL from renewable feedstocks with low volatility, such as sugars, glycerin, syngas, or methane. Chemical products produced from biobased MVL and its related derivatives could fill a need for inexpensive, renewable consumer and industrial products not based on petroleum or other nonrenewable resources. The present invention is concerned with industrially relevant compounds and polymers derived from biobased MVL and derivatives.

One aspect not in accordance with the present invention is concerned with polymer precursor compounds (aka polymer building blocks) of biobased MVL or MVL derivatives. In one or more embodiments, polymer precursor compounds of ring-opened biobased MVL or derivatives thereof are disclosed, wherein such compounds are selected from the group consisting of Formula (XXIVa), Formula (XXIVb), Formula (XXIVc), Formula (XXIVd), Formula (XXIVe), Formula (XXIVf), and combinations thereof: 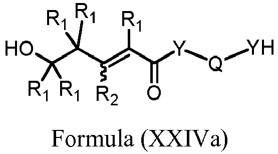 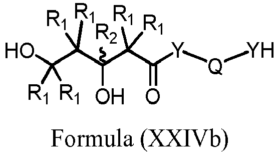 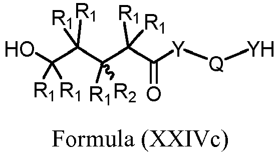 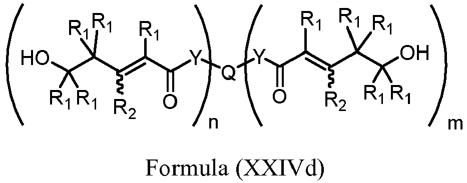 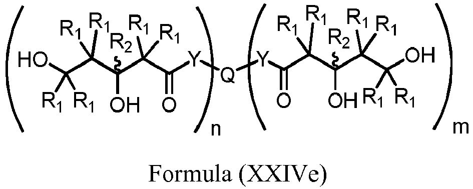 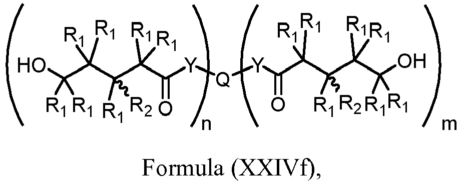 wherein, independently of each occurrence,
n is 1 to 50;
m is 1 to 50;
R₁ is independently selected from -H or an alkyl (preferably C₁-C₆ alkyls, more preferably -CH₃), with R₁ being most preferably -H;
R₂ is an alkyl (preferably C₁-C₆, more preferably -CH₃);
Y can be independently selected from -O-, -S-, -N(H), or -N(R₃);
R₃ is a linear or branched alkyl, cycloalkyl, aryl, or heteroaryl; and
Q is a single- or multiatom linkage independently selected from hydrocarbyl, substituted hydrocarbyl, heteroatom-containing hydrocarbyl, substituted heteroatom-containing hydrocarbyl, aryl, substituted aryl, heteroatom-containing aryl, and substituted heteroatom-containing aryl.

Polymers can be prepared from one or more of the foregoing ring-opened biobased MVL precursor compounds.

The invention is concerned with a biobased polymer comprising recurring monomeric units synthesized from compositions comprising one or more of the ring-opened biobased MVL compounds or polymers thereof, dispersed or dissolved in a solvent system.

In one or more embodiments, polymer precursor compounds of biobased MVL-diols or MVL-diacids are disclosed, wherein such compounds are selected from the group consisting of Formula (XXVa), Formula (XXVb), Formula (XXVc), Formula (XXVd), Formula (XXVe), Formula (XXVf), and combinations thereof: 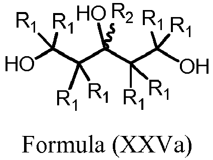 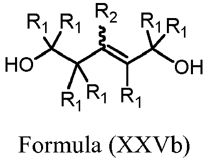 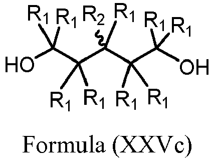 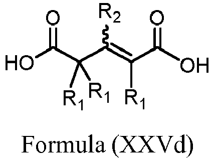 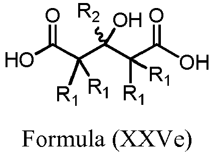 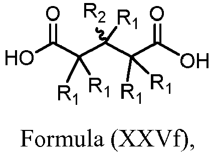 wherein, independently for each occurrence:
R₁ is independently selected from -H or an alkyl (preferably C₁-C₆ alkyls, more preferably -CH₃), with R₁ being most preferably -H; and
R₂ is an alkyl (preferably C₁-C₆, more preferably -CH₃).

Another aspect not in accordance with the present invention is also concerned with compositions comprising one or more of the biobased MVL-diol or MVL-diacid precursor compounds or polymers thereof, dispersed or dissolved in a solvent system.

In one or more embodiments, polymer precursor compounds of biobased MVL-glycidyl ether/esters are disclosed, wherein such compounds are selected from the group consisting of Formula (XVa), Formula (XVb), Formula (XVc), Formula (XVd), Formula (XVe), Formula (XVf), Formula (XVg), Formula (XVh), Formula (XVi), Formula (XVj), Formula (XVk), Formula (XVl), Formula (XVm), Formula (XVn), Formula (XVo) and combinations thereof: 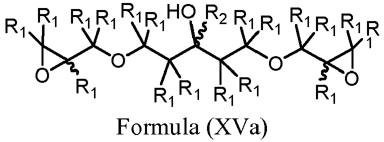 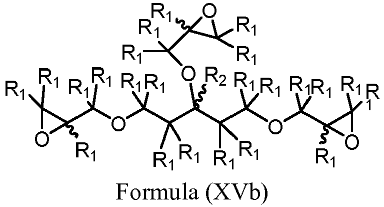 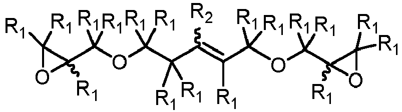 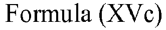 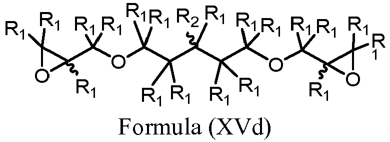 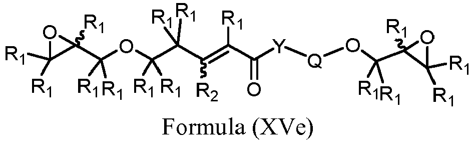 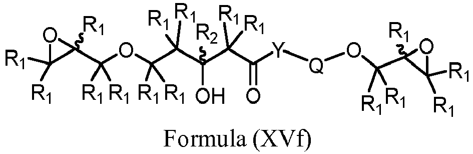 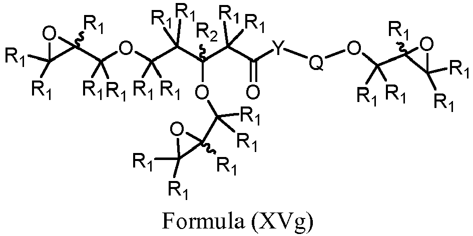 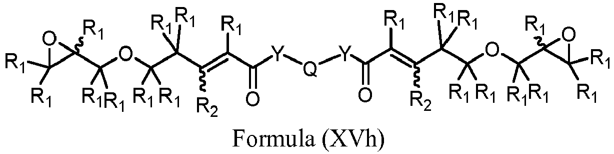 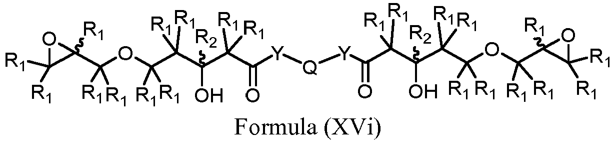 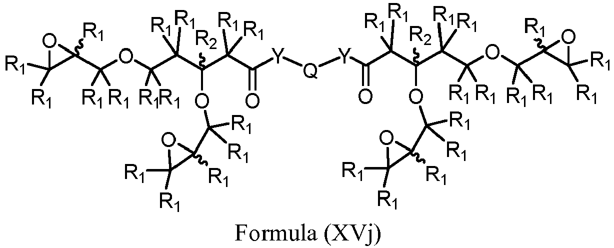 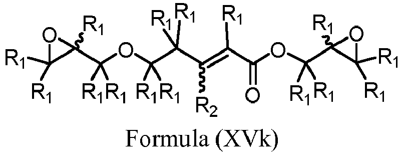 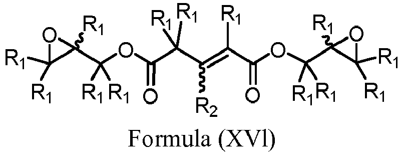 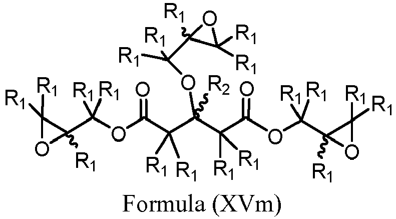 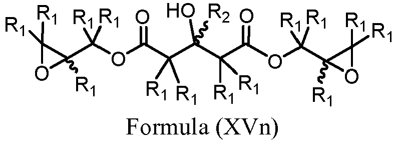 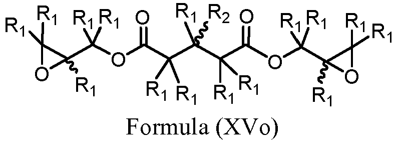 wherein, independently of each occurrence,
R₁ is independently selected from -H or an alkyl (preferably C₁-C₆ alkyls, more preferably -CH₃), with R₁ being most preferably -H;
R₂ is an alkyl (preferably C₁-C₆, more preferably -CH₃);
Q is a single- or multiatom linkage independently selected from hydrocarbyl, substituted hydrocarbyl, heteroatom-containing hydrocarbyl, substituted heteroatom-containing hydrocarbyl, aryl, substituted aryl, heteroatom-containing aryl, and substituted heteroatom-containing aryl;
Y is -O-, -S-, -N(H), or -N(R₃); and
R₃ is linear or branched alkyl, cycloalkyl, aryl, or heteroaryl.

Polymers can be prepared from one or more of the foregoing biobased MVL-glycidyl ether/ester precursor compounds.

Compositions can be provided comprising one or more of the biobased MVL-glycidyl ether/ester precursor compounds or polymers thereof, dispersed or dissolved in a solvent system.

Other biobased polymers and oligomers can be prepared from biobased MVL or derivatives as described herein, and demonstrated in the Examples.

### BRIEF DESCRIPTION OF THE DRAWINGS

Figure (Fig.) 1 illustrates reaction schemes for conversion of biobased mevalonic acid to various classes of compounds.

### DETAILED DESCRIPTION OF THE INVENTION

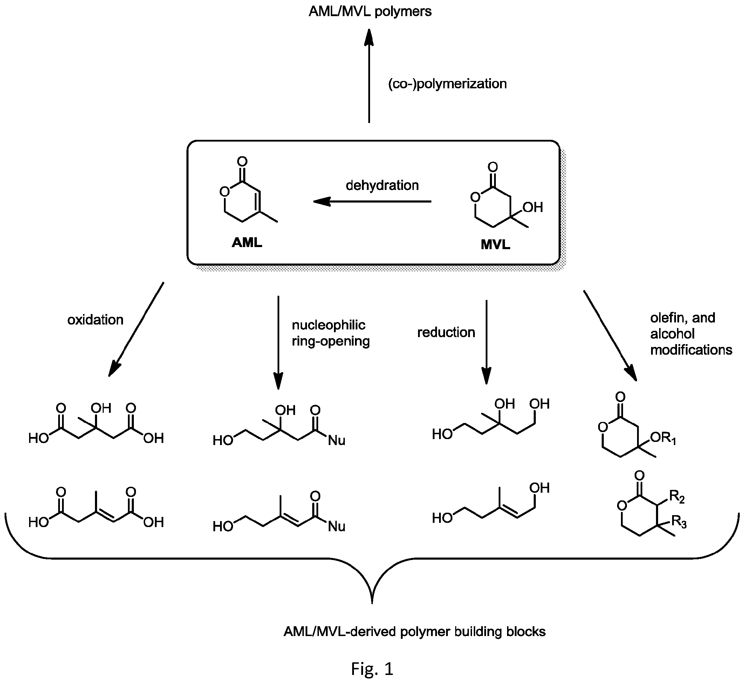

In more detail, described herein are compounds (e.g., monomers, oligomers, and/or polymers) derived from biobased compounds, and specifically biobased MVL and its related derivatives. Figure 1 provides a summary of generalized conversion pathways for biobased mevalonic acid to various classes of compounds. Through oxidation these biobased precursors can be reacted to yield building blocks for (unsaturated-) polyesters, polyester polyols and polyamides, as well as precursors for glycidyl esters and omega-alkenyl esters (e.g., allyl ethers, homoallyl ethers, vinyl ethers, etc.). Through reduction, these biobased precursors can be reacted to yield building blocks for (unsaturated-) polyesters, polyester polyols, polycarbonates, as well as precursors for glycidyl ethers and omega-alkenyl ethers. Through nucleophilic ring opening and/or amidation, these biobased precursors can be reacted to yield building blocks for polyester polyols, chain-extender for polyurethanes, or polyesters. Thus, the resulting compounds have a biobased origin, providing several advantages over petroleum-derived chemicals and compounds.

The term "biobased" as used herein means that the compound was synthesized from a biological precursor, and specifically a renewable biological carbon source, such as biomass (as opposed to a non-renewable petroleum-based carbon source). ASTM has set a method standard to calculate the level of biobased material included in a composition: ASTM D6866 - Standard Test Methods for Determining the Biobased Content of Solid, Liquid, and Gaseous Samples Using Radiocarbon Analysis. The biobased content of a composition is the amount of biobased carbon in the material as fraction weight (mass) or percent weight (mass) of the total organic carbon in the material. ASTM D6866 is similar to radiocarbon dating without the age equations. It is done by determining a ratio of the amount of radiocarbon (14C) in the material to that of a modern reference standard. The ratio is reported as a percentage referred to as percent modern carbon (units "pMC"). If the material being analyzed is a mixture of present day radiocarbon and fossil carbon (i.e., containing no radiocarbon), then the pMC value obtained correlates directly to the amount of biobased material present in the sample.

ASTM D6866 distinguishes carbon resulting from contemporary biomass-based input material from those derived from fossil-based material. Biomass contains a well-characterized amount of Carbon-14 that is easily differentiated from other materials such as fossil fuels that do not contain any Carbon-14. "Biomass" is generally defined as plant material, vegetation or agricultural waste used as a fuel or energy source. The ratio of carbon-14 isotope to carbon-12 isotope for biomass carbon is generally known to those skilled in the art to be about 2x10⁻¹² to 1 based on the current natural abundance of carbon-14 to carbon-12 as taken from air samples. Since the amount of Carbon-14 in biomass is known, a percentage of carbon from renewable sources can be calculated easily from the total organic carbon in the sample. Zero percent Carbon-14 indicates a complete lack of Carbon-14 atoms in a material, thus indicating a fossil or petroleum-based carbon source. Likewise, 100% Carbon-14 (after atmospheric correction) indicates a modern biobased carbon source.

The modern reference standard used in radiocarbon dating is a NIST (National Institute of Standards and Technology) standard with a known radiocarbon content equivalent approximately to the year AD 1950. AD 1950 was chosen since it represented a time prior to thermo-nuclear weapons testing which introduced large amounts of excess radiocarbon into the atmosphere with each explosion (termed "bomb carbon"). This was a logical point in time to use as a reference for archaeologists and geologists. For reference, an archaeologist or geologist using radiocarbon dates, AD 1950 equals "zero years old." It also represents 100 pMC. "Bomb carbon" in the atmosphere reached almost twice normal levels in 1963 at the peak of testing and prior to the treaty halting the testing. Its distribution within the atmosphere has been approximated since its appearance, showing values that are greater than 100 pMC for plants and animals living since AD 1950. It has gradually decreased over time with today's value being near 105 pMC. This means that a fresh biomass material such as corn, sugar cane or soybeans would give a radiocarbon signature near 105 pMC. By presuming ∼105 pMC represents present day biomass materials and 0 pMC represents petroleum derivatives, the measured pMC value for that material will reflect the proportions of the two component types. For example, a material derived 100% from present day soybeans would give a radiocarbon signature near 105 pMC. But if it was diluted with 50% petroleum carbon, it would give a radiocarbon signature near 53 pMC.

The "biobased content" of a material is reported as a percent value relating total renewable organic carbon to total organic carbon. The final result is calculated by multiplying the pMC value measured for the material by 0.95 (to adjust for bomb carbon effect). The final % value is cited as the mean biobased result and assumes all the components within the analyzed material were either present day living (within the last decade) or fossil in origin. In one aspect, the materials used in the invention (e.g., precursor compounds or resulting polymers) have a biobased content of greater than 0%, more preferably greater than 10%, more preferably greater than 25%, more preferably greater than 50%, and even more preferably greater than 75%. Preferably, the materials used in the invention are substantially entirely biobased according to ASTM D6866, which means they are 95 percent or more of biological origin. Thus, it will be appreciated that biobased products can be distinguished from petroleum-based products by carbon finger-printing. Thus, biobased polymers and polymeric precursors according to the invention will have a higher radioactive Carbon-14 (14C) content or a higher 14C/12C ratio than the same type of polymer of petroleum (non-renewable) origin. In one aspect, the biobased polymeric precursors and/or resulting polymers will have a 14C/12C ratio of greater than 0, preferably greater than 1.

The biobased MVL can serve as a common starting material for the production of several classes of polymers.

MVL and AML themselves are monomers for polymerization reactions. The lactone structure of MVL and AML allows for ring-opening co-polymerization. The ring-opening co-polymerization of MVL-derived β-methyl-δ-valerolactone has been reported previously. In addition to ring-opening polymerization, AML may be a co-monomer for free radical polymerization reactions. During the dehydration reaction of MVL different AML isomers, differing by the position of the unsaturation, i.e., 3,4-dehydromevalonolactone (4-methyl-3,6-dihydro-2H-pyran-2-one), 4,5-dehydromevalonolactone (4-methyl-3,4-dihydro-2H-pyran-2-one), and exo-dehydromevalonolactone (4-methylenetetrahydro-2H-pyran-2-one), can be obtained. These isomers may be utilized as monomers for ring-opening and free radical polymerization similar to AML.

In addition to the direct polymerization of MVL and related compounds described above, they can be converted into building blocks for step-growth polymers, such as diols and diacids among others. These conversions can be broadly categorized by the type of chemical reaction: i) Oxidation, ii) Reduction, iii) Lactone ring-opening, iv) Olefin modification, and v) Alcohol modification.

Full oxidation of MVL and related lactones to afford alpha,omega-dicarboxylic acids can be realized by treatment with high-valent metal oxides such as CrO₆, or KMnO₄ among others, which can also be employed in catalytic amounts in the presence of a stoichiometric strong oxidant. The oxidation of primary alcohols to carboxylic acids is also possible with nitric acid, or molecular oxygen as the oxidant. Examples of above reagents can be found for carboxylic acid as well as lactone oxidation. The MVL-derived dicarboxylic acids may find use as building blocks for polyesters, alkyd resins, unsaturated polyester resins, polyester polyols, or polyamides to name a few.

Full reduction of MVL and related lactones leads to substituted alpha,omega-diols. Metal hydrides, such as lithium tetrahydridoaluminate, elemental sodium or hydrogen in the presence of a metal catalyst can be employed to reduce the lactone functional group to two primary alcohols. These MVL-derived di-, and polyols may be used as building blocks for polyesters, alkyd resins, unsaturated polyester resins, polyester polyols, polycarbonates, or vinyl-urethane resins among others.

Nucleophiles such as alcohols, thiols, or amines are able to ring-open lactones. The reaction of MVL and related lactones with di-, or multifunctional nucleophiles results in the formation of substituted ring-opened MVL-based di-, or polyols. The nucleophiles can be either homofunctional, e.g. glycerol, or ethylene diamine among others, or heterofunctional, such as ethanolamine, or mercapto ethanol among others. The uses of these ring-opened derivatives are equivalent to the above mentioned MVL reduction products.

Modifications of the alkene group in AML and its isomers include epoxidation, dihydroxylation, Michael addition, Diels-Alder reaction, or [2+2]-cycloaddition among others. The reaction products are diverse in functionality, and possibly serve as building blocks for a variety of polymers. The lactone functionality is preserved in all the above mentioned reactions. Ring-opening polymerization of these compounds can result in the formation of modified aliphatic polyester with applications as thermoplastic elastomers, unsaturated polyester resins, or polyurethanes among others.

The tertiary alcohol in MVL can be modified to form ether and ester derivatives. Functional groups promoting polymerization, or properties such as solubility and adhesion can be introduced. It has been reported that a methacrylate with pendant MVL ester group can be obtained from MVL. It can be envisioned that this bi-functional adduct is able to participate in free-radical and ring-opening polymerization to produce acrylics and vinyl polyesters.

The MVL-derived di-, or polyols and -acids can also serve as precursors for epoxy resins, poly(vinylether)s, or multifunctional cyclic carbonates.

Several of the primary but not exclusive polymer classes that can be produced utilizing MVL/AML or MVL/AML-derived building blocks, and their applications are described in the following:

### Unsaturated polyester resins

Unsaturated polyester resins are typically prepared by polyesterification reactions that take place in heated, stirred reactor vessels fitted with packed columns, steam-jacketed condensers and water condensers. Production may be carried out under continuous or semi-continuous (batchwise) esterification processes. The component raw materials which are the inputs, or starting materials, for esterification processes used to produce unsaturated polyester resins include (with abbreviations in parentheses):
(i) a saturated dibasic acid component,
(ii) an unsaturated dibasic acid component,
(iii) a glycol component,
(iv) a hydrocarbon modifier component.

The saturated dibasic acid component may comprise: phthalic anhydride (PA), isophthalic acid (IPA), chlorendic anhydride, adipic acid, or terephthalic acid, among others.

The unsaturated dibasic acid component may comprise: maleic anhydride (MAN) or fumaric acid, among others.

The glycol component may comprise: propylene glycol (PG), diethylene glycol (DEG), dipropylene glycol (DPG), ethylene glycol (EG), or neopentyl glycol (NPG), among others.

The hydrocarbon modifier component may comprise: cyclopentadiene (CP), dimethylcyclopentadiene, dicyclopentadiene (DCPD), and other plasticizers, among others.

Biobased MVL and related derivatives could be utilized in the production of UPRs in place of, or in addition to an unsaturated dibasic acid component or glycol component.

UPRs may be classified in ways including: i) the content of the phthalic or isophthalic components in the UPR; ii) the commercial type (or usage), such as spray-up, press-molding, chemical-resistant, or fire-retardant (each requiring a specific UPR composition) and iii) the method of application, (for both reinforced plastic or non-reinforced plastics), relating to how a UPR is used along with other components to create coatings or solid objects, among others.

### Phthalic and isophthalic unsaturated polyester resins

Phthalic resins may be prepared in one stage cooks with all ingredients charged at once. Typical phthalic-maleic resins (in a 1:1 ratio) require about 1-14 hours at 150-250°C, depending upon the molecular weight distribution of the resulting polymer product.

Isophthalic resins may be prepared in two stages: 1) an aromatic acid and glycol are reacted until the theoretically correct (calculated) amount of water has been collected and an acid number of 10-15 has been reached; 2) the material from 1) is then cooled to below 170°C and an unsaturated acid is charged. This process can require as much as 22-32 hours at 180-230°C, depending upon the molecular weight distribution of the resulting polymer product. Esterification catalysts are sometimes used in amounts of 0.02-0.05% of the total charge.

When the polycondensation reaction described above is completed (as determined by the acid number and by the viscosity of a specimen sample dissolved in styrene or another organic solvent), the resin is cooled to about 165°C or less.

Hydroquinone (HQ) or a similar compound may be added as an inhibitor and the mixture then thinned with styrene, which is inhibited with HQ, toluhydroquinone (THQ) or mono-tert-butyl hydroquinone. t-Butyl catechol and para-benzoquinone may also be used as inhibitors.

All of the above steps are typically carried out under a blanket of nitrogen, carbon dioxide or an inert gas. The removal of water produced during the reaction is facilitated by a slow stream of inert gas, passed through the system.

A biobased MVL-based isophthalic resin might be prepared by partial and/or full substitution of 1) maleic anhydride with AML-derived unsaturated diacid, and/or 2) glycol (i.e. propanediol) with AML-derived diol, to yield materials for use in gel coats, chemical-resistant isophthalic and press-molding applications.

### Commercial unsaturated polyester resin types

Spray-up resins may typically be applied to an open mold, by spraying the resin mixture (comprising unsaturated polyester + a diluent such as styrene), along with a catalyst, directly onto a prepared surface. Chopped glass, or other materials such as fibers, may first be layered upon the surface to be sprayed, or mixed within the resin mixture, and then sprayed together in combination. A typical formulation might comprise 1.0-2.0 moles of phthalic anhydride, 1.0 mole of maleic anhydride, 2.1-2.8 moles of propylene glycol and 35-45% styrene.

DCPD-based resins are well-utilized due to their lower cost and lower styrene content. In marine applications, DCPD-type resins represent over 75% of the total market. Bathroom applications of spray-up resins utilize, almost entirely, the DCPD variety. Their main disadvantage is their relatively low resistance to both oxygen and ultraviolet light. A typical formulation for a DCPD-type resin might comprise 0.8 mole of DCPD, 1.0 mole of maleic anhydride, 0.4 mole of ethylene glycol, 0.2 mole of diethylene glycol and 30-35% styrene.

Flame-retardant resins are typically utilized for interior construction, with their use controlled by building codes and regulations. Flame resistance is achieved by adding: aluminum trihydrate, either alone or in combination with certain phosphate compounds (e.g., dimethyl methyl phosphonate (DMMP)), chlorendic anhydride, tetrabromophthalic anhydride, dibromoneopentyl glycol and tetrabromobisphenol A. Flame-retardant resins based on brominated compounds are the most common.

Chemical-resistance is achieved in UPRs by the composition of acids and glycols used. Isophthalic acid and terephthalic acid are often used in resins that require a high level of hydrolytic stability and chemical resistance. Neopentyl glycol is used to impart good water-resistant properties to gel coats. Trimethylpentanediol is sometimes used when a higher degree of corrosion resistance is required. Cyclohexanedimethanol is sometimes used to impart both outdoor durability and corrosion resistance. High-performance applications that require specific chemical resistance and/or temperature resistance typically utilize premium-grade vinyl ester resins, bisphenol Abased resins, and chlorendic anhydride-based resins.

Press-molding resins are typically comprised of precursors in the ratio of about 3.0 moles of maleic anhydride to about 1.0 mole of an aromatic acid, with a corresponding amount of propylene glycol. In some instances, an aromatic acid is not used. Certain press-molding resins can be combined with thermoplastic additives to promote a low profile (i.e., a smooth surface) and reduce shrinkage (low-shrinkage properties permit the production of large parts with high dimensional accuracy). Such low-profile/low-shrink molding resins are usually neat (non-copolymerized) maleic anhydride-propylene glycol polyesters containing 30-40% styrene. The thermoplastic additives are usually proprietary mixtures, typically 30-50% solutions of thermoplastic resins dissolved in styrene or monomer mixtures that are primarily styrene. Typically, the resins are copolymers of styrene, vinyl acetate or other monomers. Systems comprised of 60% UPR and 40% thermoplastic additive are often used for the molding of automotive parts, which demand relatively stringent surface requirements. Systems comprised of 70% UPR and 30% thermoplastic additive are typically used for electrical equipment housing and other similar parts. Low-profile/low-shrink resins are used for press-molding resin materials such as sheet-molding compound (SMC) and bulk-molding compound (BMC). SMC is used in applications requiring electrical- or corrosion-resistance, and is often formulated in the ratio of 1.0 mole of isophthalic acid to 3.0 moles of maleic anhydride. Glass and other fillers are also added to increase strength. Such thickened resin systems are also used in some BMC applications.

An example of a biobased MVL-based press-molding resin is an unsaturated polyester obtained from biobased AML-derived diacid, and -diol. A similar unsaturated polyester partially composed of maleic anhydride or propylene glycol blocks may also be used. As described above, these materials are usually used with thermoplastic additives.

### Unsaturated polyester resin application methods

Fiberglass-reinforced plastics (FRP) are used for structural applications. A liquid resin is combined with the required additives (e.g., promoters, accelerators, flame retardants, ultraviolet absorbers and thixotropic agents) and/or fillers and pigments before it is placed into contact with the glass reinforcement and catalyst. The neat resin-to-glass ratio is typically 65:35, while a highly-filled resin-to-glass ratio is usually 40:40:20. For filament winding and pultrusion applications, the ratio can be as low as 30:70. Catalysts are added in 0.8-2 part(s) per 100 parts of resin and are activated by heat or curing. For room temperature curing, methyl ethyl ketone peroxide is used as a catalyst in conjunction with an accelerator such as cobalt naphthenate, cobalt octoate, dimethyl aniline (DMA) or dimethylacetoacetamide (DMAA). Other catalysts include: t-butyl peroxybenzoate (TBPB), which is used in SMC and BMC materials; mixtures of benzoyl peroxide and cumene hydroperoxide (CHP) in a 2:1 molar ratio; and mixtures of CHP and either benzyl trimethylammonium chloride (BTMAC) or TBPB/t-butyl peroxyoctoate (TBPO). The curing process proceeds in two stages: the first is a soft-gel stage, which can be interrupted by cooling and resumed later; followed by a second stage which occurs upon continued heating, when an exothermic reaction begins and heating must be controlled. FRP can be used in a manufacturing process known as contact molding to manufacture large objects such as boats and storage tanks. Further manufacturing processes include press molding and injection molding. Injection molding uses BMC for the production of small parts, with high throughput and precision.

Non-reinforced plastics are based on fluid UPRs, where their formulations have a high diluent content in order to achieve fluidity. Fillers used in such UPRs might include clays and calcium carbonate, among others. Such non-reinforced UPRs are used in manufacturing processes including casting, and may also be used as: putty and filler materials (for automobile repair); potting and encapsulating compounds and surface coating resins. Surface coating resins are methyl methacrylate (MMA)-based monomer polyester resins (where MMA substitutes fully or partially for styrene), and may be cured using conventional peroxide catalysts, while special UPRs, with acrylate functional groups, may be cured by using ultraviolet light.

The physicochemical properties, and uses, of UPRs will depend upon the molar proportions of the raw materials which were used in the polyesterification processes. The average raw material requirements for different types of unsaturated polyester resins, used for a variety of applications, might include (molar ratios):

**General-Purpose Spray-Up**

| | |
|---|---|
| Conventional Ortho-Based: | 1.5 PA; 1.2 MAN; 2.2 PG; 0.5 DEG. |
| DCPD-Based: | 0.8 DCPD; 1.0 MAN; 0.4 EG; 0.2 DEG. |
| Press Molding: | 0.2 PA; 1.2 IPA; 3.7 MAN; 2.5 PG; 1.5 DEG; 0.2 DPG; 0.5 NPG |

**Chemical-Resistant**

| | |
|---|---|
| Isophthalic: | 1.0 IPA; 1.0 MAN; 2.0 PG. |
| Gel Coat: | 1.5 IPA; 1.0 MAN; 1.5 PG; 2.5 NPG. |
| Non-FRP: | 3.5 PA; 2.0 MAN; 2.0 PG; 3.0 DEG; 0.5 DPG |

### Exemplary Polymers

In the chemical structures depicted herein, a wavy line perpendicular to a chemical bond: 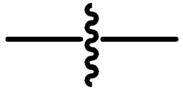 is used to indicate that the structure shown is a smaller piece (moiety) of a larger chemical entity, and accordingly, the wavy line perpendicular to a chemical bond denotes the point of chemical attachment between two chemical entities, such as a point of chemical attachment of the depicted structure to the rest of a polymer backbone or other chemical fragments in the polymer chain.

However, a bond itself drawn as a wavy line denotes a chemical bond indicating the presence of stereochemical isomers in either pure or mixed form. For example, the formula 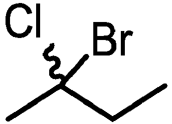 represents (R)-2-bromo-2-chlorobutane, (S)-2-bromo-2-chlorobutane, or a mixture thereof. In another example, the formula 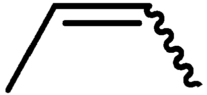 represents (Z)-2-butene, (E)-2-butene, or a mixture thereof.

In one aspect, the present invention relates to a polyester polymer synthesized using biobased MVL and comprising recurring monomeric units containing respective moieties. In one or more embodiments, the polyester comprises one or more moieties represented by Formula (Ia), Formula (Ib), Formula (Ic), Formula (Id), Formula (Ie), Formula (If), and/or Formula (Ig). In one or more embodiments, the polyester polymer comprises a plurality of moieties represented by Formula (Ia), Formula (Ib), Formula (Ic), Formula (Id), Formula (Ie), Formula (If), and/or Formula (Ig): 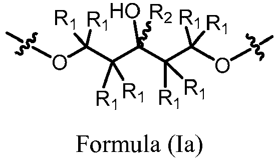 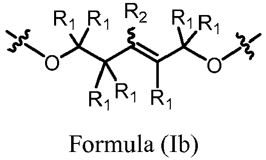 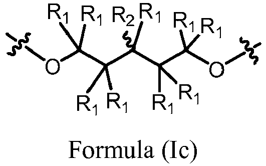 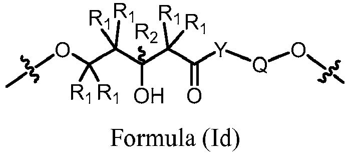 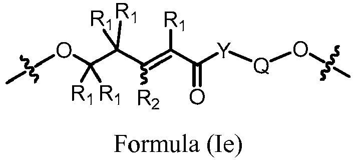 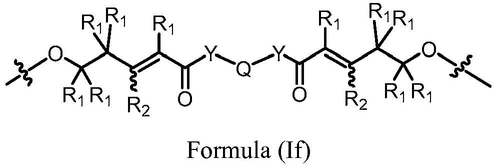 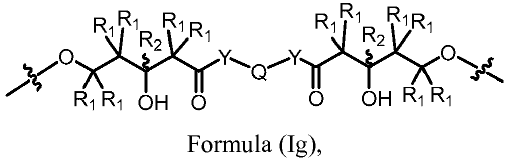 wherein, independently for each occurrence:
R₁ is independently selected from H or C₁-C₆ alkyl;
R₂ is C₁-C₆ alkyl;
Y is -O-, -S-, -N(H), or -N(R₃);
R₃ is C₁-C₆ alkyl, cycloalkyl, or aryl; and
Q is a single- or multiatom linkage independently selected from hydrocarbyl, substituted hydrocarbyl, heteroatom-containing hydrocarbyl, substituted heteroatom-containing hydrocarbyl, aryl, substituted aryl, heteroatom-containing aryl, and substituted heteroatom-containing aryl.

In a second aspect, the present invention relates to a polyester comprising one or more moieties represented by Formula (IIa), Formula (IIb), and/or Formula (IIc): 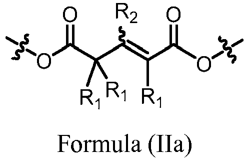 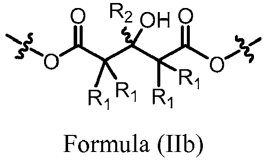 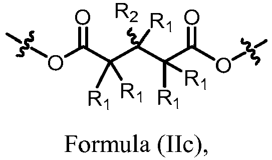 wherein, independently for each occurrence,
R₁ is independently selected from H or C₁-C₆ alkyl; and
R₂ is a C₁-C₆ alkyl.

In certain embodiments, the present invention relates to any of the aforementioned polyester polymers, further comprising a monomeric, oligomeric, or polymeric repeat unit (block) represented by the Formula (III) 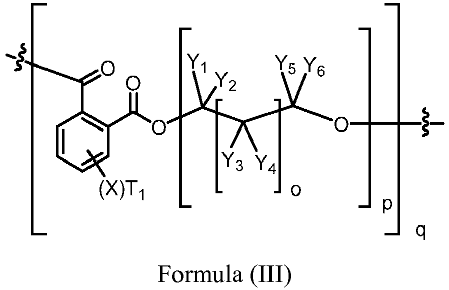 wherein, independently for each occurrence,
X is halogen or C₁-C₆ alkyl;
T1 is 0, 1, 2, 3 or 4;
Y₁, Y₂, Y₃, Y₄, Y₅ and Y₆ are independently H or C₁-C₆ alkyl;
o is 0, 1, 2, 3 or 4;
p is an integer from 1 to 50; and
q is an integer from 2 to 100,000.

In an additional embodiment, the present invention relates to the aforementioned polyester block copolymer, further comprising a monomeric, oligomeric, or polymeric repeat unit (block) represented by Formula (IV): 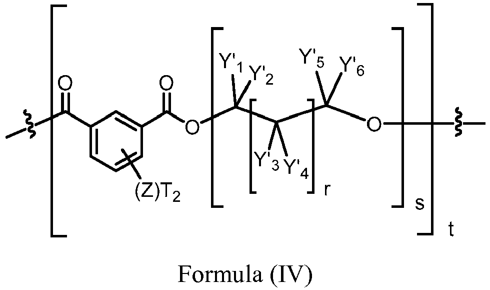 wherein, independently for each occurrence,
Z is halogen or C₁-C₆ alkyl;
T2 is 0, 1, 2, 3 or 4;
Y'₁, Y'₂, Y'₃, Y'₄, Y'₅ and Y'₆ are independently H or C₁-C₆ alkyl;
r is 0, 1, 2, 3 or 4;
s is an integer from 1 to 50; and
t is an integer from 2 to 100,000.

In an additional embodiment, the present invention relates to the aforementioned polyester block copolymer, further comprising a monomeric, oligomeric, or polymeric repeat unit (block) represented by Formula (V) 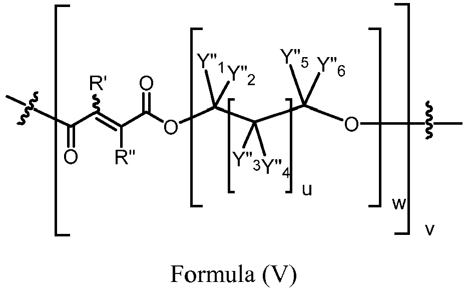 wherein, independently for each occurrence,
R' and R" are independently H or C₁-C₆ alkyl;
Y"₁, Y"₂, Y"₃, Y"₄, Y"₅ and Y"₆ are independently H or C₁-C₆ alkyl;
u is 0, 1, 2, 3 or 4;
w is an integer from 1 to 50; and
v is an integer from 2 to 100,000.

In an additional embodiment, the present invention relates to any of the aforementioned polyester block copolymers, further comprising a moiety represented by Formula (VI): 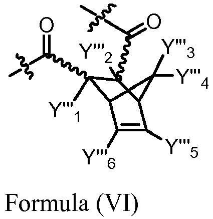 wherein, independently for each occurrence,
Y'''₁, Y'''₂, Y'''₃, Y'''₄, Y'''₅ and Y'''₆ are independently H or C₁-C₆ alkyl.

In an additional embodiment, the present invention relates to any of the aforementioned polyester block copolymers, further comprising a moiety represented by Formula (XXVII): 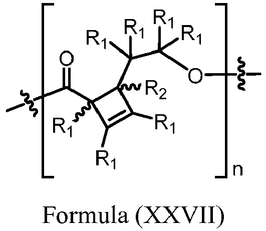 wherein, independently for each occurrence,
R₁ is independently selected from H or C₁-C₆ alkyl;
R₂ is a C₁-C₆ alkyl; and
n is an integer from 1 to 100,000.

In certain embodiments, the present invention relates to monomeric, oligomeric, or polymeric repeat units represented by the Formula (VIII): 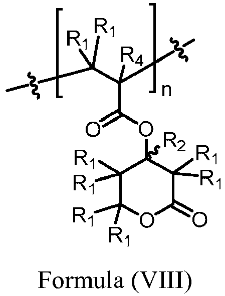 wherein, independently of each occurrence,
R₁, and R₄ are independently selected from H or C₁-C₆ alkyl;
R₂ is C₁-C₆ alkyl; and
n is an integer from 2 to 100,000.

In certain embodiments, the present invention relates to the aforementioned oligomeric, or polymeric repeat units represented by the Formula (VIII) further comprising another monomeric, oligomeric, or polymeric repeat unit (block) represented by the Formula (IX): 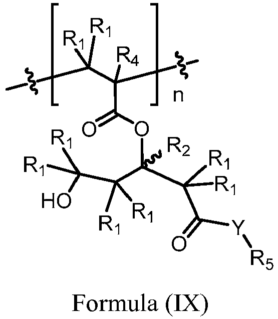 wherein, independently of each occurrence,
R₁, and R₄ are independently selected from H or C₁-C₆ alkyl;
R₂ is C₁-C₆ alkyl;
n is an integer from 1 - 10,000;
R₅ is hydrogen, linear or branched alkyl, cycloalkyl, aryl, or heteroaryl;
R₃ is linear or branched C₁-C₆ alkyl, cycloalkyl, aryl, or heteroaryl; and
Y is -O-, -S-, -N(H), or -N(R₃).

In certain embodiments, the present invention relates to the aforementioned polymer blocks further comprising another monomeric, oligomeric, or polymeric repeat unit (block) represented by the Formula (X): 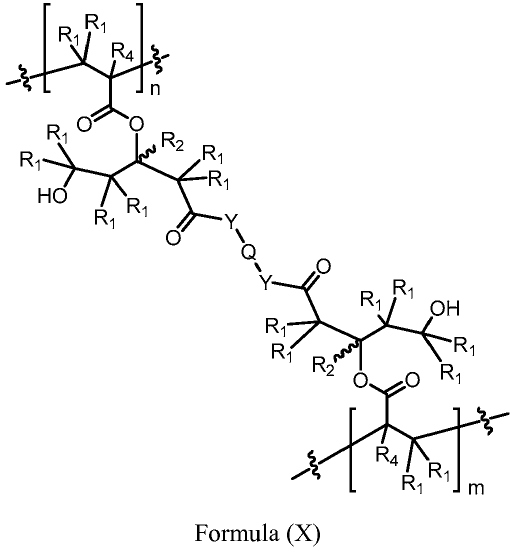 wherein, independently of each occurrence,
R₁, R₄, are independently selected from H or C₁-C₆ alkyl;
R₂ is C₁-C₆ alkyl;
R₃ is C₁-C₆ alkyl, cycloalkyl, or aryl;
Y is -O-, -S-, -N(H), or -N(R₃); and
Q is a single- or multiatom linkage independently selected from hydrocarbyl, substituted hydrocarbyl, heteroatom-containing hydrocarbyl, substituted heteroatom-containing hydrocarbyl, aryl, substituted aryl, heteroatom-containing aryl, and substituted heteroatom-containing aryl.

In certain embodiments, the present invention relates to any one of the aforementioned block copolymers, further comprising a block of poly(isoprenyl acetate).

In certain embodiments, the present invention relates to any one of the aforementioned block copolymers, further comprising a block of poly(ethylene) or poly(propylene).

In certain embodiments, the present invention relates to any one of the aforementioned block copolymers, further comprising a block of poly(vinylalcohol) or poly(vinylester).

In certain embodiments, the present invention relates to any one of the aforementioned block copolymers, further comprising a block of poly(acrylic acid) or poly(methyl acrylate).

In certain embodiments, the present invention relates to any one of the aforementioned block copolymers, further comprising a block of poly(methacrylic acid) or poly(methyl methacrylate).

In certain embodiments, the present invention relates to any one of the aforementioned block copolymers, further comprising a block of poly(acrylamide) or poly(methacrylamide).

In certain embodiments, the present invention relates to any one of the aforementioned block copolymers, further comprising a block of polystyrene.

In certain embodiments, the present invention relates to any one of the aforementioned block copolymers, wherein R₁ is H; and R₂ is -CH₃.

In certain embodiments, the present invention relates to any one of the aforementioned block copolymers, wherein R₄ is -CH₃.

In certain embodiments, the present invention relates to any one of the aforementioned block copolymers, wherein Y is -N(H), and Q is ethyl, n-propyl, n-butyl, n-pentyl, or n-hexyl.

In certain embodiments, the present invention relates to any one of the aforementioned block copolymers, wherein Y is -O-.

In certain embodiments, the present invention relates to any one of the aforementioned block copolymers, wherein T1 is 0.

In certain embodiments, the present invention relates to any one of the aforementioned block copolymers, wherein T2 is 0.

In certain embodiments, the present invention relates to any one of the aforementioned block copolymers, wherein Y₁, Y₂, Y₃, Y₄, Y₅ and Y₆ are H.

In certain embodiments, the present invention relates to any one of the aforementioned block copolymers, wherein Y₁, Y₂, Y₅ and Y₆ are H; and Y₃ and Y₄ are -CH₃.

In certain embodiments, the present invention relates to any one of the aforementioned block copolymers, wherein Y₁, Y₂, Y₄, Y₅ and Y₆ are H; and Y₃ is -CH₃.

In certain embodiments, the present invention relates to any one of the aforementioned block copolymers, wherein Y₁ or Y₅ is -CH₃.

In certain embodiments, the present invention relates to any one of the aforementioned block copolymers, wherein Y'₁, Y'₂, Y'₃, Y'₄, Y'₅ and Y'₆ are H.

In certain embodiments, the present invention relates to any one of the aforementioned block copolymers, wherein Y'₁, Y'₂, Y'₅ and Y'₆ are H; and Y'₃ and Y'₄ are -CH₃.

In certain embodiments, the present invention relates to any one of the aforementioned block copolymers, wherein Y'₁, Y'₂, Y'₄, Y'₅ and Y'₆ are H; and Y'₃ is -CH₃.

In certain embodiments, the present invention relates to any one of the aforementioned block copolymers, wherein Y'₁ or Y'₅ is -CH₃.

In certain embodiments, the present invention relates to any one of the aforementioned block copolymers, wherein Y"₁, Y"₂, Y"₃, Y"₄, Y"₅ and Y"₆ are H.

In certain embodiments, the present invention relates to any one of the aforementioned block copolymers, wherein Y"₁, Y"₂, Y"₅ and Y"₆ are H; and Y"₃ and Y"₄ are -CH₃.

In certain embodiments, the present invention relates to any one of the aforementioned block copolymers, wherein Y"₁, Y"₂, Y"₄, Y"₅ and Y"₆ are H; and Y"₃ is -CH₃.

In certain embodiments, the present invention relates to any one of the aforementioned block copolymers, wherein Y"₁ or Y"₅ is -CH₃.

In certain embodiments, the present invention relates to any one of the aforementioned block copolymers, wherein Y'''₁, Y'''₂, Y'''₃, Y'''₄, Y'''₅ and Y'''₆ are H.

In certain embodiments, the present invention relates to any one of the aforementioned block copolymers, wherein p is 1 or 2.

In certain embodiments, the present invention relates to any one of the aforementioned block copolymers, wherein s is 1 or 2.

In certain embodiments, the present invention relates to any one of the aforementioned block copolymers, wherein o is 0 or 1.

In certain embodiments, the present invention relates to any one of the aforementioned block copolymers, wherein o is 0.

In certain embodiments, the present invention relates to any one of the aforementioned block copolymers, wherein r is 0 or 1.

In certain embodiments, the present invention relates to any one of the aforementioned block copolymers, wherein r is 0.

In certain embodiments, the present invention relates to any one of the aforementioned block copolymers, wherein u is 0 or 1.

In certain embodiments, the present invention relates to any one of the aforementioned block copolymers, wherein u is 0.

### Polyurethanes

Polyurethanes are typically prepared from the following component raw materials:
(i) A polyol component,
(ii) a di- and/or poly-isocyanate component,
(iii) a cross-linker (or chain-extender) component, and
(iv) a blowing agent component.

The polyol component may comprise polyethers, such as: poly (propylene glycol), poly (ethylene glycol), poly (propylene glycol - co - ethylene glycol), or poly (tetramethylene glycol), among others. The polyol component may also comprise polyesters derived from adipic acid, phthalic acid, diethylene glycol, and 1,2-propylene glycol among others. Their hydroxy functionality is typically around 2, and their average molecular weights are between 200 to 10,000 grams per mol.

The di- and/or poly-isocyanate component may comprise: toluene diisocyanate (TDI), hexamethylene diisocyanate (HDI), methylene diphenyl diisocyanate (MDI), triphenylmethane triisocyanate among others.

The cross-linker (or chain-extender) may comprise: glycerol, trimethanol propane, ethylene glycol, ethylene diamine and phenylene diamine among others.

A blowing agent component may comprise: pentane, carbon dioxide, methylene chloride, water among others.

Biobased MVL, and related derivatives, can be utilized in place of, or in addition to, a polyol and/or chain-extender, in the production of polyurethanes.

Polyurethanes can be characterized by their chemical structure of a thermoplastic or thermoset, and their physical appearance of a rigid solid, a soft elastomer, or a foam. They are used in commercial applications categorized as flexible slab (furniture, bedding, automotive among others), flexible molded foams (automotive seating, bedding among others), rigid foams (appliances, insulation, automotive among others), solid elastomers (coatings, elastomers, adhesives, medical among others), reaction injection molding (RIM) (mechanical, automotive among others), carpet backing (unitary, attached cushion among others), and two-component formulations (casting, encapsulation, sealants among others).

Polyurethanes may also be characterized by their method of preparation: i) Solvent free, ii) in solution, or iii) water-borne dispersions.

### Polyurethane dispersions

Polyurethane dispersions are aqueous dispersions of high molecular weight polyurethanes, or poly(urethane-urea), that are stabilized by ionic (i.e. anionic, or cationic) or hydrophilic nonionic groups in the polyurethane backbone. Characteristics of the final application include adhesion, solvent and abrasion resistance, flexibility and toughness.

An aqueous anionic polyurethane dispersion may be prepared by the solvent method. The di- or polyisocyanate, di- or polyol, and acid diol in a typical molar ratio of 4:2:1, and solvent are added to a stirred vessel. Acid diols typically contain a carboxylic or sulfonic acid group, such as dimethylol propanoic acid, or dimethylol butanoic acid. As solvents, acetone, or N-methyl pyrrolidone may be employed among others. To this mixture is typically added 0.1 mol% catalyst, such as dibutyl tin dilaureate (DBTDL). Other catalysts such as triethylenediamine (DABCO), dimethylethanolamine, stannous octoate, or bismuth carboxylates may also be used. The polyurethane prepolymer is then formed at elevated temperatures of 50 - 100 °C until a desired isocyanate content of typically 0.1 to 10 % is reached. After cooling the mixture to below 90 °C, the solution is neutralized by the addition of base, such as triethylamine, and addition of water to form the dispersion. The polyurethane can then be chain-extended by the further addition of a diamine chain-extender such as ethylene diamine.

In a variation, a prepolymer solution may be formed by the reaction of di- or polyisocyanate, and di- or polyol in a molar ratio between 1.2 and 2.5 in acetone. At temperatures below 50 °C, addition of a chain extender, typically a sulfonate functional diamine such as 1,1-diaminomethanesulfonic acid, 2-[(2-aminoethyl)amino]ethanesulfonic acid, or 1,1-diaminopropanesulfonic acid among others introduces the water-solubilizing/dispersing group. Addition of water and base produces the anionic aqueous polymer dispersion.

Removal of the acetone, or N-methyl pyrrolidone solvent may be necessary and is affected by distillation to produce the polyurethane dispersions with 30 - 50 % solids content. Solids contents of up to 60 % are preferred for textile coatings and dipping applications.

Applications of polyurethane dispersions are coatings for wood floors, plastics, or as automotive base coats, finishes for wood, fiberglass, or leather, and adhesives.

For leather finishes, after extension, the polyurethane dispersion prepared as described above can be thickened with the addition of a polymeric thickener, chosen between the polyurethanes, or acrylic polymers, or derivatives of modified polysaccharides. They comprise typically between 0.1 and 7% by weight of the dispersion. In addition, pigments, flame retardants, and feel additives may be used as well. The impregnation of the fabric can be conducted by any suitable method known in the art. Examples include dipping, spraying or doctor blading. After impregnating, the impregnated textile may have excess resin removed to leave the desired amount of dispersion within the textile. Typically, this is accomplished by passing the impregnated textile through rubber rollers.

### Polyurethane foams

Polyurethane foams are typically produced in solvent-free processes. Common uses for flexible foams are upholstered furniture, mattresses, automotive seating among others.

A 80:20 isomeric mixture of 2,4-, and 2,6-toluene diisocyanate (TDI) is commonly used in forming flexible and semiflexible foams, whereas modified TDI, MDI or poly phenyl diisocyanates are generally chosen for rigid foams. For flexible foams, the diisocyanate is reacted with a polyether polyol, typically based on poly ethylene oxide, or poly propylene oxide, with a reactive hydroxy number between 2 and 3, in the presence of a blowing agent, and/or auxiliary blowing agent. A low boiling gas, such as trichlorofluoronate, and/or water may be added to provide the foaming. Other auxiliary blowing agents are methylene chloride, n-pentane, cyclopentane, and acetone amongst others.

Polyurethane foams are commonly prepared by the one-shot method. A typical formulation of a one-shot flexible foam is 100 parts of polyether polyol (typically a mixture of diol and triol), 40 parts toluene diisocyanate, 4 parts water, 1 part surfactant, 1 part amine catalyst, and 0.5 parts tin catalyst. Commonly used surfactants are silicone based polymers and copolymers. Stannous oleate, stannous octoate, and dibutyl tin dilaureate may be used as tin catalysts, and DABCO, triethylamine, *N*-ethyl morpholine, and *N,N,N',N'*-tetramethyl-1,3-butane diamine may be used as amine catalysts.

In continuous slab production the above mentioned chemical components are delivered at fixed rates to a mixing head that moves across a conveyer into a continuous mold, usually formed by the conveyer and adjustable sideboards, at outputs of 100 - 200 lb/min. Temperature curing may be executed in a curing oven until track-free (15 min to 24 h). Typically, the slab stock is further processed by cut-off saws, horizontal and vertical trimmers, slicing machines, and hot-wire cutters among others. Centrifugal peeling, and horizontal table splitting may be used to obtain flexible slab products for mattresses, cushions, pillows, sponges, shock-absorbing materials and others.

Biobased MVL-based polyurethanes can be exemplified by partial of full substitution of the polyols by MVL-derived polyester polyols, and by partial or full substitution of chain-extenders by MVL-derived diols and/or triols.

### Exemplary Polymers

In another aspect, the present invention relates to a polyurethane polymer synthesized using biobased MVL and comprising recurring monomeric units containing respective moieties. In one or more embodiments, the polyurethane comprises one or more moieties represented by Formula (Ia), Formula (Ib), Formula (Ic), Formula (Id), Formula (Ie), Formula (If), and/or Formula (Ig), as defined above. In one or more embodiments, the polyurethane polymer comprises a plurality of moieties represented by Formula (Ia), Formula (Ib), Formula (Ic), Formula (Id), Formula (Ie), Formula (If), and/or Formula (Ig), as defined above.

In certain embodiments, the present invention relates to the aforementioned polyurethane polymer, further comprising a second monomeric, oligomeric, or polymeric repeat unit (block) represented by Formula (XI): 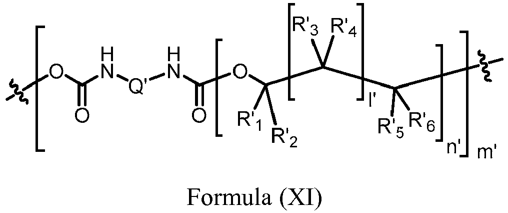 wherein, independently of each occurrence,
R'₁, R'₂, R'₃, R'₄, R'₅ and R'₆ are independently H or C₁-C₆ alkyl;
l' is 0, 1, 2, 3, or 4;
n' is an integer from 2 - 1,000;
m' is an integer from 1 - 100,000; and
Q' is a single- or multiatom linkage independently selected from hydrocarbyl, substituted hydrocarbyl, heteroatom-containing hydrocarbyl, substituted heteroatom-containing hydrocarbyl, aryl, substituted aryl, heteroatom-containing aryl, and substituted heteroatom-containing aryl.

In certain embodiments, the present invention relates to any one of the aforementioned block copolymers, wherein R'₁, R'₂, R'₃, R'₄, R'₅ and R'₆ is H, and l' is 2.

In certain embodiments, the present invention relates to any one of the aforementioned block copolymers, wherein R'₁, or R'₅ is CH₃, and l' is 0.

In certain embodiments, the present invention relates to any one of the aforementioned block copolymers, wherein R'₁, R'₂, R'₃, R'₄, R'₅ and R'₆ is H, and l' is 0.

In certain embodiments, the present invention relates to any of the aforementioned polyurethane blocks, further comprising a third monomeric, oligomeric, or polymeric repeat unit (block) represented by Formula (XII): 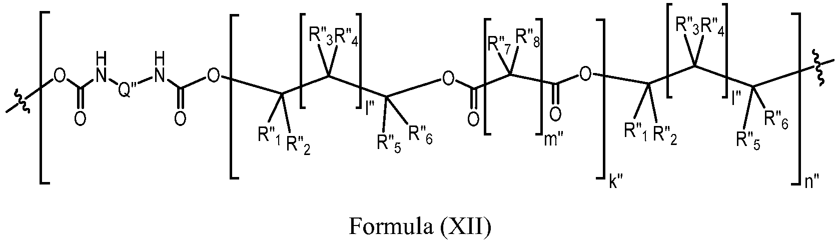 wherein, independently of each occurrence,
R"₁, R"₂, R"₃, R"₄, R"₅, R"₆, R"₇, and R"s are independently H or C₁-C₆ alkyl;
n" is an integer from 2 - 100,000;
m" is an integer from 0 - 4;
l" is an integer from 0 - 4;
k" is an integer from 2 - 1,000; and
Q" is a single- or multiatom linkage independently selected from hydrocarbyl, substituted hydrocarbyl, heteroatom-containing hydrocarbyl, substituted heteroatom-containing hydrocarbyl, aryl, substituted aryl, heteroatom-containing aryl, and substituted heteroatom-containing aryl.

In certain embodiments, the present invention relates to any one of the aforementioned block copolymers, wherein R"₁, R"₂, R"₃, R"₄, R"₅, R"₆, R"₇ and R"s is H, l" is 0 or 2, and m" is 4.

In certain embodiments, the present invention relates to any of the aforementioned polyurethane blocks, further comprising a third monomeric, oligomeric, or polymeric repeat unit (block) represented by Formula (XIII): 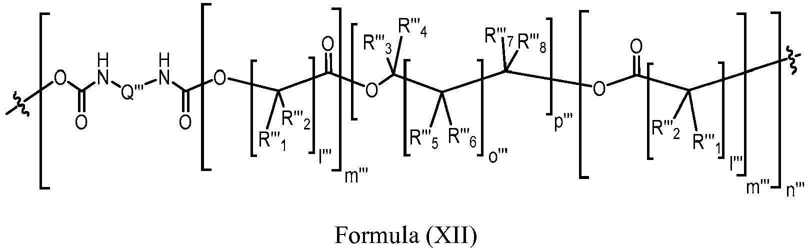 wherein, independently of each occurrence,
R'''₁, R'''₂, R'''₃, R'''₄, R'''₅, R'''₆, R'''₇, and R'''₈ are independently H or C₁-C₆ alkyl;
n''' is an integer from 2 - 100,000;
m''' is an integer from 0 - 100;
l''' is an integer from 1 - 6;
o''' is an integer from 0 - 6;
p''' is an integer from 1 - 100; and
Q''' is a single- or multiatom linkage independently selected from hydrocarbyl, substituted hydrocarbyl, heteroatom-containing hydrocarbyl, substituted heteroatom-containing hydrocarbyl, aryl, substituted aryl, heteroatom-containing aryl, and substituted heteroatom-containing aryl.

In certain embodiments, the present invention relates to any one of the aforementioned polyurethane block copolymers, wherein R'''₁, R'''₂, R'''₃, R'''₄, R'''₅, R'''₆, R'''₇ and R'''₈ is H, l" is 5, o''' is 0, and p''' is 1 or 2.

In certain embodiments, the present invention relates to any one of the aforementioned polyurethane block copolymers, wherein R'''₁, R'''₂, R'''₃, R'''₄, R'''₅, R'''₆, R'''₇ and R'''₈ is H, l" is 5, o''' is 2 or 4, and p''' is 1.

In certain embodiments, the present invention relates to any one of the aforementioned polyurethane block copolymers, wherein R'''₅, and R'''₆ is CH₃, l" is 5, o''' is 1, and p''' is 1.

In certain embodiments, the present invention relates to any one of the aforementioned polyurethane copolymers, wherein Q', Q", and/or Q''' are comprised of a structure represented by Formula (XIIIa), Formula (XIIIb), Formula (XIIIc), Formula (XIIId), Formula (XIIIe), Formula (XIIIf), Formula (XIIIg), Formula (XIIIh), and/or Formula (XIIIi):

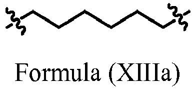

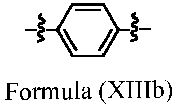

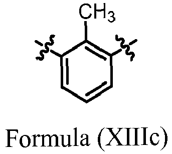

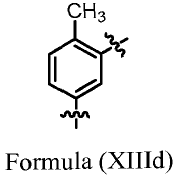

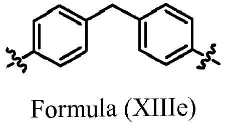

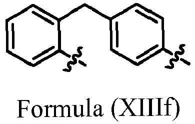

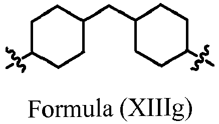

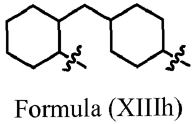

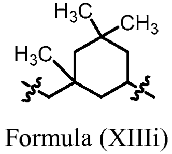

In certain embodiments, the present invention relates to any of the aforementioned polyurethane blocks, further comprising another monomeric, oligomeric, or polymeric repeat unit (block) represented by Formula (XIVa), Formula (XIVb), and/or Formula (XIVc): 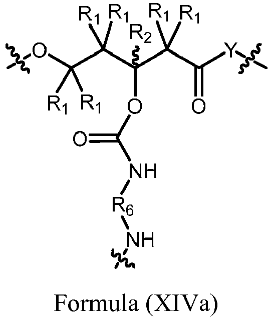 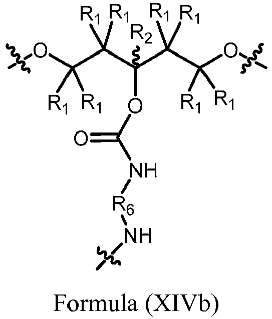 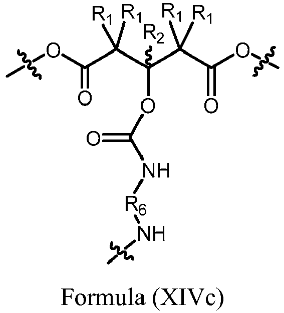 wherein, independently of each occurrence,
R₁ are independently selected from H or C₁-C₆ alkyl;
R₂ is a C₁-C₆ alkyl;
Y is -O-, -S-, -N(H), or -N(R₃);
R₃ is linear or branched alkyl, cycloalkyl, aryl, or heteroaryl; and
R₆ is linear or branched alkyl, cycloalkyl, aryl, or heteroaryl. It is preferably represented by Formula (XIIIa), Formula (XIIIb), Formula (XIIIc), Formula (XIIId), Formula (XIIIe), Formula (XIIIf), and/or Formula (XIIIg)

In certain embodiments, the present invention relates to any one of the aforementioned polyurethane block copolymers, wherein R₁ are H, and R₂ is CH₃.

In certain embodiments, the present invention relates to any one of the aforementioned polyurethane block copolymers, wherein R₁ are H, R₂ is CH₃, and Y is -N(H).

### Glycidyl ethers

Epoxies are an important class of thermoset plastics. They are typically formulated from a di- and/or polyepoxide resin, which may be diluted with reactive and/or non-reactive fillers, and are cured by a hardener. Curing may also be achieved by light or temperature.

The resin component are low to high molecular weight molecules that contain at least two epoxide groups. They may include bisphenol A diglycidyl ether, bisphenol F diglycidyl ether, and phenol novolac polyglydicyl ethers among others. Reactive diluents or reactive fillers are aliphatic mono- and di-glydicyl ethers such dodecanol glycidyl ether, 2-ethyl hexyl glycidyl ether, or neopentyl glycol diglycidyl ether among others.

The hardener component may be chosen from one of the following classes: i) Polyfunctional nucleophiles (e.g. diethylene triamine, triethylene tetramine, tetraethylene pentamine, piperidine, menthanediamine, or polysulfides among others), ii) Cyclic anhydrides (e.g. phthalic anhydride, hexahydrophthalic anhydride, pyromellitic anhydride, maleic anhydride among others), or iii) Lewis acid or base catalysts.

The curing with nucleophilic hardeners may occur in the ambient, while anhydride and Lewis acid or base catalyzed curing takes place at elevated temperature of 80 - 150 °C.

### Exemplary Glycidyl Ethers

In another aspect, the present invention relates to a chemical molecule (non-polymeric compound) synthesized using biobased MVL, represented by Formula (XVa), Formula (XVb), Formula (XVc), Formula (XVd), Formula (XVe), Formula (XVf), Formula (XVg), Formula (XVh), Formula (XVi), Formula (XVj), Formula (XVk), Formula (XVl), Formula (XVm), Formula (XVn), and/or Formula (XVo): 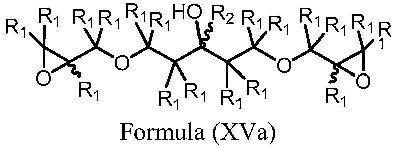 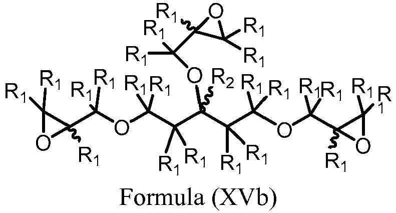 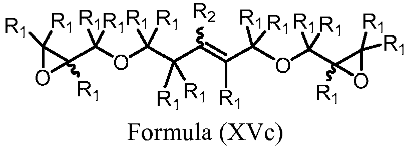 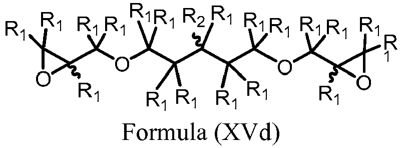 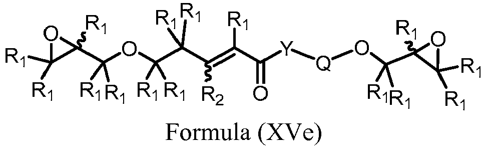 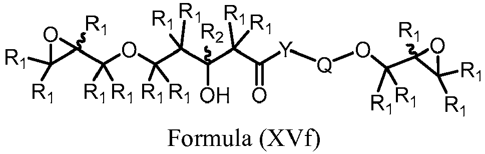 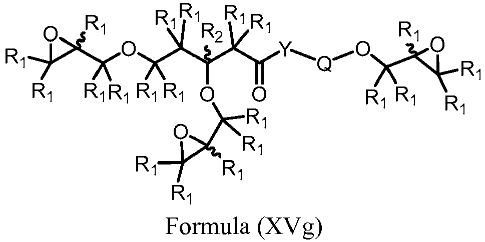 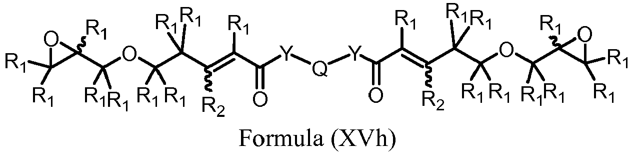 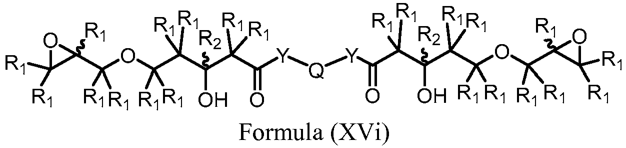 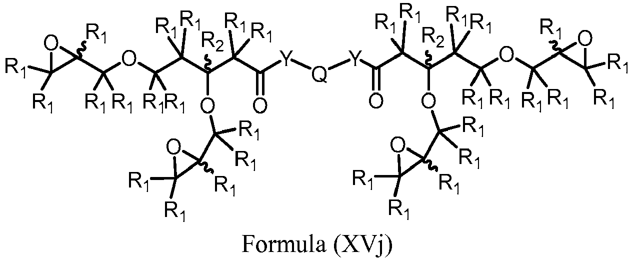 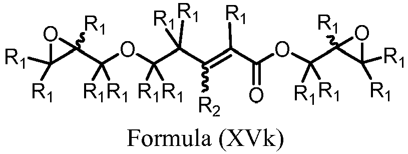 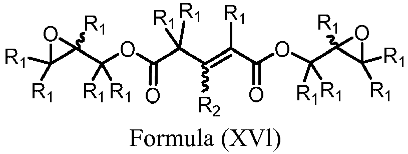 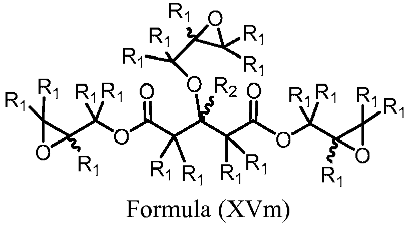 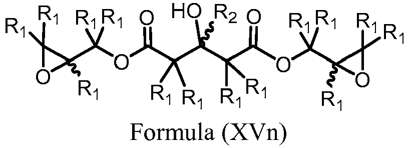 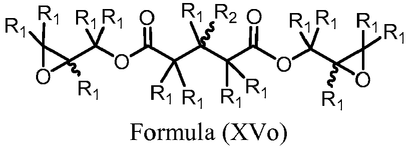 wherein, independently of each occurrence,
R₁ is independently selected from H or C₁-C₆ alkyl;
R₂ is a C₁-C₆ alkyl;
Q is a single- or multiatom linkage independently selected from hydrocarbyl, substituted hydrocarbyl, heteroatom-containing hydrocarbyl, substituted heteroatom-containing hydrocarbyl, aryl, substituted aryl, heteroatom-containing aryl, and substituted heteroatom-containing aryl;
Y is -O-, -S-, -N(H), or -N(R₃); and
R₃ is linear or branched alkyl, cycloalkyl, aryl, or heteroaryl.

In another aspect, the present invention relates to a chemical molecule (non-polymeric compound) synthesized using biobased AML, and represented by Formula (XXVI): 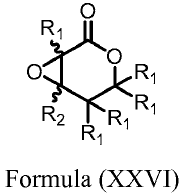 wherein, independently of each occurrence,
R₁ is independently selected from H or C₁-C₆ alkyl; and
R₂ is a C₁-C₆ alkyl.
The invention also related to polymers derived from the foregoing non-polymeric glycidyl ether/ester and epoxide compounds.

### Exemplary omega-alkenyl ethers and esters

In another aspect, the present invention relates to a chemical molecule (non-polymeric compound) synthesized using biobased MVL, and represented by Formula (XVIa), Formula (XVIb), Formula (XVIc), Formula (XVId), Formula (XVIe), Formula (XVIf), Formula (XVIg), Formula (XVIh), Formula (XVIi), Formula (XVIj), Formula (XVIk), Formula (XVII), Formula (XVIm), Formula (XVIn), and/or Formula (XVIo): 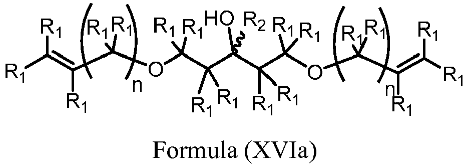 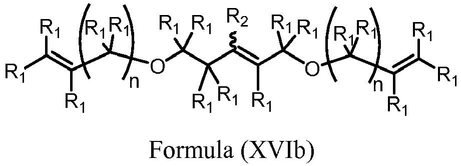 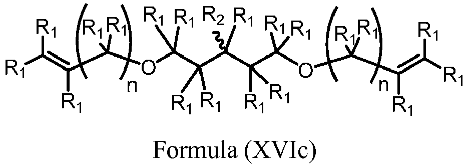 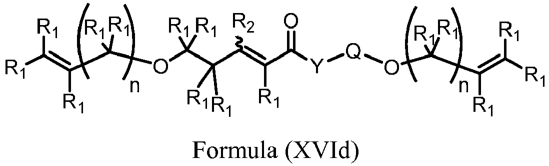 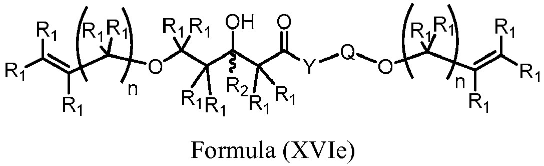 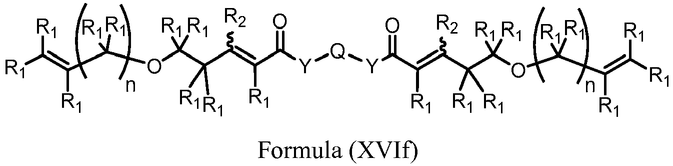 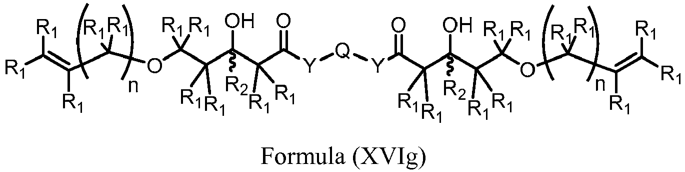 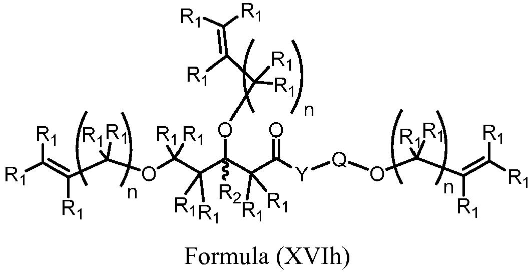 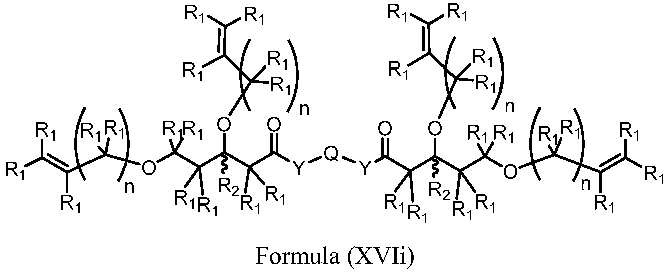 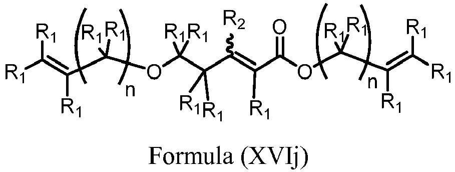 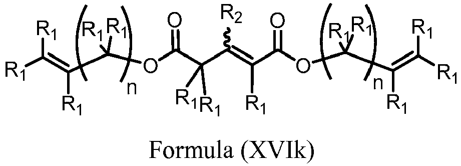 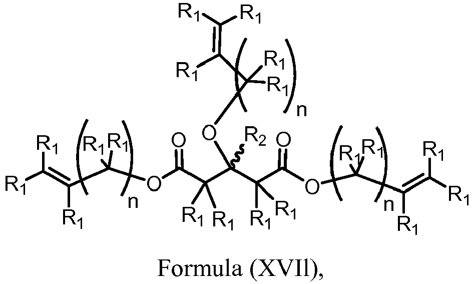 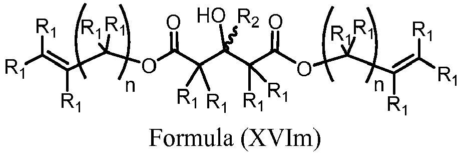 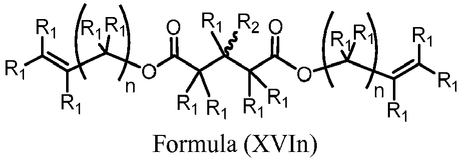 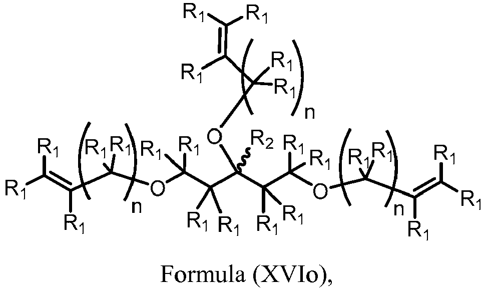 wherein, independently of each occurrence,
R₁ is independently selected from H or C₁-C₆ alkyl;
R₂ is a C₁-C₆ alkyl;
n is an integer from 0 to 50;
Q is a single- or multiatom linkage independently selected from hydrocarbyl, substituted hydrocarbyl, heteroatom-containing hydrocarbyl, substituted heteroatom-containing hydrocarbyl, aryl, substituted aryl, heteroatom-containing aryl, and substituted heteroatom-containing aryl;
Y is -O-, -S-, -N(H), or -N(R₃); and
R₃ is linear or branched alkyl, cycloalkyl, aryl, or heteroaryl.
The invention also related to polymers derived from the foregoing non-polymeric omega-alkenyl ether and ester compounds.

### Exemplary cyclic carbonates

In another aspect, the present invention relates to a chemical molecule (non-polymeric compound) synthesized using biobased MVL, represented by Formula (XVIIa), Formula (XVIIb), Formula (XVIIc), Formula (XVIId), Formula (XVIIe), Formula (XVIIf), and/or Formula (XVIIg): 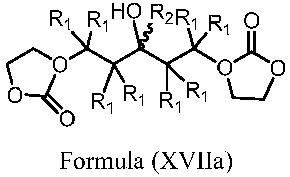 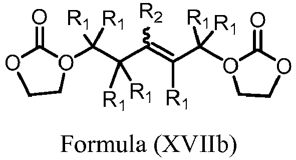 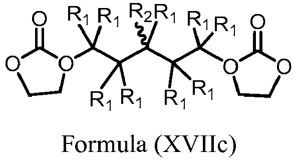 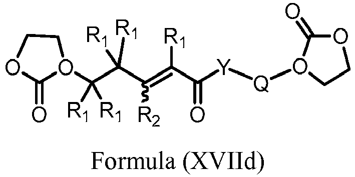 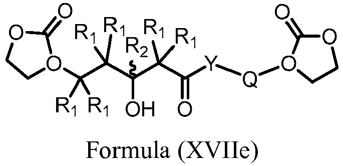 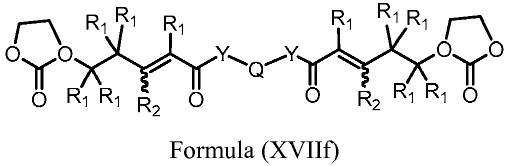 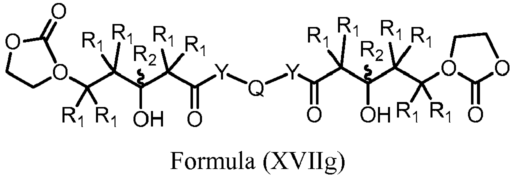 wherein, independently of each occurrence,
R₁ are independently selected from H or C₁-C₆ alkyl;
R₂ is a C₁-C₆ alkyl;
Y is -O-, -S-, -N(H), or -N(R₃);
R₃ is alkyl, cycloalkyl, or aryl; and
Q is a single- or multiatom linkage independently selected from hydrocarbyl, substituted hydrocarbyl, heteroatom-containing hydrocarbyl, substituted heteroatom-containing hydrocarbyl, aryl, substituted aryl, heteroatom-containing aryl, and substituted heteroatom-containing aryl. The invention also related to polymers derived from the foregoing non-polymeric cyclic carbonate compounds.

### Exemplary polycarbonates

In another aspect, the present invention relates to a polymer comprising recurring monomers represented by Formula (XVIIIa), Formula (XVIIIb), Formula (XVIIIc), Formula (XVIIId), Formula (XVIIIe), Formula (XVIIIf), and/or Formula (XVIIIg): 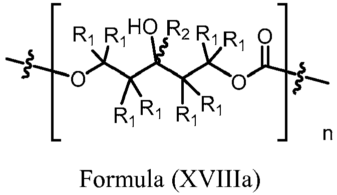 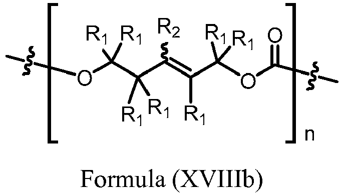 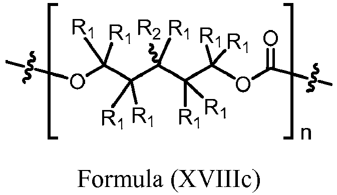 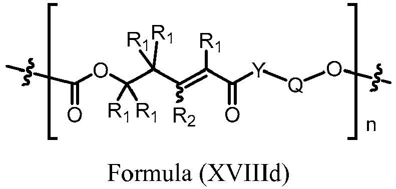 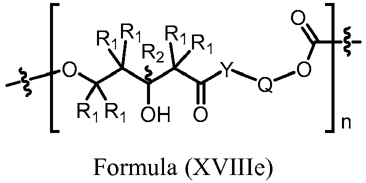 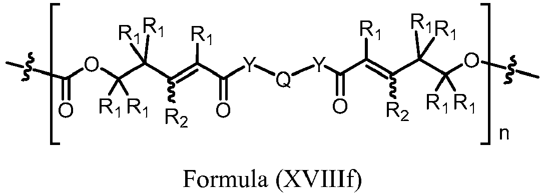 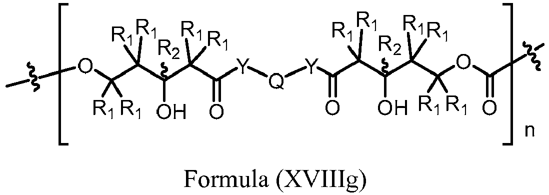 wherein, independently of each occurrence,
R₁ is independently H or C₁-C₆ alkyl;
R₂ is a C₁-C₆ alkyl;
Y is -O-, -S-, -N(H), or -N(R₃);
R₃ is C₁-C₆ alkyl, cycloalkyl, or aryl;
n is an integer from 2 to 100,000; and
Q is a single- or multiatom linkage independently selected from hydrocarbyl, substituted hydrocarbyl, heteroatom-containing hydrocarbyl, substituted heteroatom-containing hydrocarbyl, aryl, substituted aryl, heteroatom-containing aryl, and substituted heteroatom-containing aryl.

In another embodiment, the present invention relates to the aforementioned polycarbonate, further comprising a second monomeric, oligomeric, or polymeric repeat unit (block) represented by Formula (XIX): 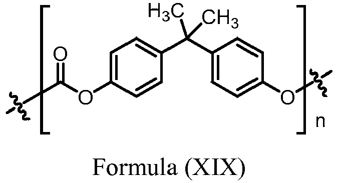 wherein n is an integer from 1 to 100,000.

### Exemplary polyamides

In one aspect, the present invention relates to a polyamide polymer synthesized using biobased MVL and comprising recurring monomeric units containing respective moieties. In one or more embodiments, the polyamide comprises one or more moieties represented by Formula (XXa), Formula (XXb), and/or Formula (XXc): 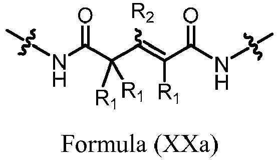 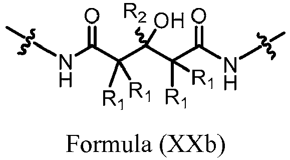 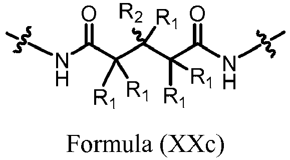 wherein, independently for each occurrence,
R₁ is independently H or C₁-C₆ alkyl; and
R₂ is C₁-C₆ alkyl.

In another embodiment, the present invention relates to the aforementioned polyamide polymer further comprising a second monomeric, oligomeric, or polymeric repeat unit (block) represented by Formula (XXI): 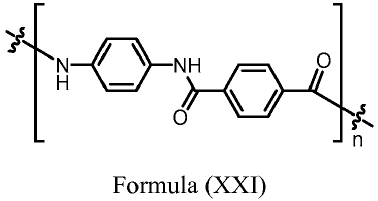 wherein n is an integer from 1 to 100,100.

In another embodiment, the present invention relates to the aforementioned polyamide polymer further comprising a third monomeric, oligomeric, or polymeric repeat unit (block) represented by Formula (XXII): 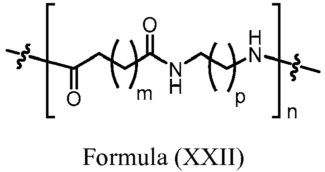 wherein n is an integer from 1 to 100,100;
m is an integer from 1 to 50; and
p is an integer from 1 to 50.

In another embodiment, the present invention relates to the aforementioned polyamide polymer further comprising a fourth monomeric, oligomeric, or polymeric repeat unit (block) represented by Formula (XXIII): 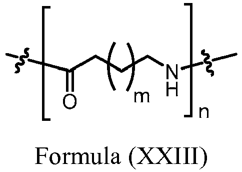 wherein n is an integer from 1 to 100,100; and
m is an integer from 0 to 50.

Biobased compositions are also described herein. The compositions comprise one or more of the foregoing biobased MVL precursor compounds, derivatives, and/or polymers thereof dispersed or dissolved in a suitable solvent system. Such compositions would have various uses depending upon the particular precursor compound or polymer used, as discussed in part above, and advantageously be based upon renewable materials, instead of being derived principally from petroleum (non-renewable) sources.

Additional advantages of the various embodiments of the invention will be apparent to those skilled in the art upon review of the disclosure herein and the working examples below. It will be appreciated that the various embodiments described herein are not necessarily mutually exclusive unless otherwise indicated herein. For example, a feature described or depicted in one embodiment may also be included in other embodiments, but is not necessarily included. Thus, the present invention encompasses a variety of combinations and/or integrations of the specific embodiments described herein.

As used herein, the phrase "and/or," when used in a list of two or more items, means that any one of the listed items can be employed by itself or any combination of two or more of the listed items can be employed. For example, if a composition is described as containing or excluding components A, B, and/or C, the composition can contain or exclude A alone; B alone; C alone; A and B in combination; A and C in combination; B and C in combination; or A, B, and C in combination.

The present description also uses numerical ranges to quantify certain parameters relating to various embodiments of the invention. It should be understood that when numerical ranges are provided, such ranges are to be construed as providing literal support for claim limitations that only recite the lower value of the range as well as claim limitations that only recite the upper value of the range. For example, a disclosed numerical range of about 10 to about 100 provides literal support for a claim reciting "greater than about 10" (with no upper bounds) and a claim reciting "less than about 100" (with no lower bounds).

### EXAMPLES

The following examples set forth methods and compounds in accordance with the invention. It is to be understood, however, that these examples are provided by way of illustration and nothing therein should be taken as a limitation upon the overall scope of the invention.

### Synthesis of Biobased MVL derived compounds

### Example 1 - Synthesis of 3,5-dihydroxy-N-(2-hydroxyethyl)-3-methylpentanamide (MVLea)

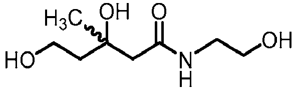

This compound is represented by Formula (Id), (XIVa), (XVf), (XVIe), (XVIIe), (XVIIIe), and (XXIVb). Biobased (R)-(-)-Mevalonolactone (3.30 g, 25.4 mmol) was dissolved in equal amounts of THF by volume, and 1.30 g (21.3 mmol) ethanolamine were added dropwise, leading to a pale yellow solution. The reaction was stirred for 12 h, and then precipitated into 40 mL diethylether. The supernatant was decanted off and the residue was washed four times with 20 mL diethylether, before being dried *in vacuo* overnight to constant weight, yielding 4.02 g of a light yellow oil (98 %). ¹H NMR (500 MHz, DMSO) δ 7.95 (t, *J* = 5.2 Hz, 1H), 4.94 (s, 1H), 4.69 (s, 1H), 4.39 (s, 1H), 3.52 (dd, *J* = 11.6, 7.1 Hz, 2H), 3.39 (t*, J* = 6.0 Hz, 2H), 3.13 (q, *J* = 6.0 Hz, 2H), 2.22 (s, 2H), 1.61 (td, *J* = 7.1, 2.9 Hz, 2H), 1.10 (s, 3H). ¹H NMR (500 MHz, MeOD) 8 4.67 (s, 3H), 3.52 (dd, *J* = 13.9, 7.1 Hz, 2H), 3.38 (t, *J* = 5.7 Hz, 2H), 3.16 - 2.96 (m, 2H), 2.18 (q, *J* = 14.1 Hz, 2H), 1.57 (td, *J* = 6.9, 5.4 Hz, 2H), 1.04 (s, 3H). ¹³C NMR (126 MHz, MeOD) δ 174.4, 72.1, 61.5, 59.2, 48.0, 44.4, 42.8, 27.5.

### Example 2 - Synthesis of 3,5-dihydroxy-N-(5-hydroxypentyl)-3-methylpentanamide (MVLpa)

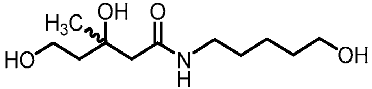

This compound is represented by Formula (Id), (XIVa), (XVf), (XVIe), (XVIIe), (XVIIIe), and (XXIVb). Biobased (R)-(-)-Mevalonolactone (1.1 g, 8.5 mmol) was dissolved in equal amounts of THF by volume, and 0.79 g (5.9 mmol) 5-amino-1-pentanol were added dropwise, leading to a pale yellow solution. The reaction was stirred for 12 h, and then precipitated into 40 mL diethylether. The supernatant was decanted off and the residue was washed four times with 20 mL diethylether, before being dried *in vacuo* overnight to constant weight, yielding 1.28 g of a light yellow oil (93% yield). ¹H NMR (500 MHz, DMSO) δ 7.92 (t, *J* = 5.5 Hz, 1H), 4.97 (s, 1H), 4.40 (s, 2H), 3.64 - 3.46 (m, 2H), 3.37 (t, *J* = 6.7 Hz, 2H), 3.04 (dd, *J* = 12.7, 6.9 Hz, 2H), 2.20 (s, 2H), 1.60 (td, *J* = 6.9, 2.0 Hz, 2H), 1.47 - 1.33 (m, 4H), 1.32 - 1.23 (m, 2H), 1.10 (s, 3H).

### Example 3 - Synthesis of N,N'-(hexane-1,6-diyl)bis(3,5-dihydroxy-3-methylpentanamide) (hmMVL₂)

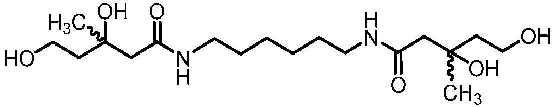

This compound is represented by Formula (Ig), (XVi), (XVj), and (XVIe), (XVIIg), (XVIIIg), (XVIf), (XVIg), (XIVa), and (XXIVe). Biobased (R)-(-)-Mevalonolactone (1.1 g, 8.5 mmol) was dissolved in equal amounts of THF by volume, and 0.45 g (3.9 mmol) 1,6-hexanediamine were added dropwise, leading to a pale yellow solution. The reaction was stirred for 12 h, and then precipitated into 40 mL diethylether. The supernatant was decanted off and the residue was washed four times with 20 mL diethylether, before being dried *in vacuo* overnight to constant weight, yielding 1.41 g of a highly viscous pale yellow oil (96 % yield). ¹H NMR (500 MHz, DMSO) δ 7.92 (t, *J* = 5.5 Hz, 2H), 4.96 (s, 2H), 4.40 (s, 2H), 3.60 - 3.47 (m, 4H), 3.34 (s, 2H), 3.03 (dd, *J* = 12.7, 6.8 Hz, 4H), 2.20 (s, 4H), 1.60 (td, *J* = 6.8, 1.9 Hz, 4H), 1.41 - 1.34 (m, 4H), 1.28 - 1.22 (m, 4H), 1.10 (s, 6H).

### Example 4 - Synthesis of 3-methylpent-2-ene-1,5-diol (AMLdiol)

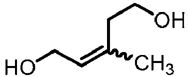

This compound is represented by Formula (Ib), (XVc), (XVIb), (XVIIb), (XVIIIb), and (XXVb). Synthesized according to: A general stereoselective synthesis of olefins; J. W. Cornforth, R. H. Cornforth, K. K. Mathew J. Chem. Soc., 1959, 112 - 127, and briefly described herein.

To a flame-dried flask was added 100 mL anhydrous ether, followed by 2.7 g lithium aluminum hydride under a nitrogen blanket. 8 g of biobased AML were added dropwise, and the mixture was stirred for 16 h at room temperature, followed by 4 h under reflux conditions. The reaction was quenched by the addition of 10 mL ethyl acetate in 10 mL diethyl ether, followed by 20 mL saturated ammonium chloride solution. The residue after filtration was extracted with 100 mL hot methanol which was then evaporated, and that residue was extracted with 3 x 30 mL diethyl ether. The combined ether phases were evaporated, and the residue distilled twice to give 2.8 g of the title compound as colorless oil. ¹H NMR (500 MHz, DMSO) δ 5.39 - 5.23 (m, 1H), 4.50 (s, 2H), 3.91 (dd, *J* = 6.7, 0.8 Hz, 2H), 3.41 (t, *J* = 7.1 Hz, 2H), 2.16 (t, *J* = 7.1 Hz, 2H), 1.68 (dd, *J* = 2.4, 1.1 Hz, 3H).

Other stereoisomers of *(Z)-3-methylpent-2-ene-1,5-diol,* i.e. *(E)-3-methylpent-2-ene-1,5-diol,* or *3-methylenepentane-1,5-diol,* or a mixture of two or three of the compounds can be obtained by dehydration of the *3-methylpentane-1,3,5-triol* mentioned in example 5. Different reaction conditions such as temperature, solvent, concentration, and the nature of catalytic acids and/or bases, influence the dehydration mechanism and can be used to change the product mixture composition.

### Example 5 - Synthesis of 3-methylpentane-1,3,5-triol (MVLtriol)

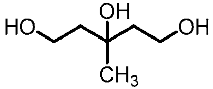

This compound is represented by Formula (Ia), (XIVb), (XVa), (XVb), (XXVa), (XVIa), (XVIIa), and (XVIIIa). Synthesized according to previous techniques: The Chemical Synthesis of Mevalonic Acid 5-Phosphate, Isopentenyl Pyrophosphate, and Related Compounds. C. D. Foote, F. Wold Biochemistry, 1963, 2 (6), 1254 - 1258. This compound incorporated into polymers is represented by Formula (Ia). It can be used in unsaturated polyester resins (examples 9-16), polyesterols for polyurethanes, or normal polyesters. It can also be used as a precursor for diglycidyl ethers, i.e. Formula (XVa).

To an oven-dried two-neck flask with 30 mL lithium aluminum hydride (1M in THF, 1.3 equivalents) in 50 mL dry THF was slowly added a solution of 3 g biobased MVL (23.1 mmol) in 5 mL dry THF leading to formation of white precipitate. The solution was heated to reflux for 18 h. The reaction was quenched by the slow addition of 100 mL water at 0 °C, and the inorganic salts filtered off. The filter cake was washed with 100 mL ethanol, and the combined organic phases were evaporated to dryness. Kugelrohr distillation of the yellow syrup resulted in 2.1 g of colorless oil. ¹H NMR (500 MHz, DMSO) δ 4.33 (t, *J* = 5.0 Hz, 2H), 4.28 (s, 1H), 3.51 (dt, *J* = 7.3, 5.0 Hz, 4H), 1.55 (dd, *J* = 7.6, 6.6 Hz, 4H), 1.06 (s, 3H).

### Example 6 - Synthesis of 3-methylpentane-1,5-diol (MVLdiol) (prophetic)

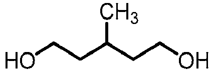

This compound is represented by Formula (Ic), (XVd), (XVId), (XVIIc), (XVIIIc), and (XXVc). In a typical experiment, 2.00 g of biobased AML, 1 mol% [Rh(acac) (CO)₂], and 1 mol% [Mo(CO)₆] were dissolved in 40 g 1,4-dioxane in a Schlenk tube and transferred into an evacuated 300-mL stainless steel autoclave. The vessel was pressurized with 120 bar hydrogen and heated up to 200 °C within 20 min leading to a hydrogen pressure of 150 bar at reaction temperature. The reaction time of 2 h was taken after accelerating the stirrer to 700 rpm. After the reaction mixture was cooled to room temperature the solvent was evaporated. The product was purified by vacuum distillation. (Adapted from: Bimetallic-Catalyzed Reduction of Carboxylic Acids and Lactones to Alcohols and Diols. A. Behr, V. A. Brehme Adv. Synth. Catal. 2002, 344, 525 - 532.)

### Example 7 - Synthesis of 3-methylpent-2-enedioic acid (AMLdiacid) (prophetic)

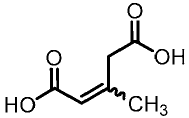

This compound is represented by Formula (IIa), (XVl), (XVII), (XXa), and (XXVb). Biobased AML (5 g) was slowly added to 20 mL nitric acid (70 %) and stirred for 4 h at 50 °C. All volatiles were distilled off, and the residue recrystallized from n-butyl acetate.

Similarly to *(Z)-3-methylpent-2-ene-1,5-diol* (Example 4), other stereoisomers, i.e. *(E)-3-methylpent-2-enedioic acid,* or *3-methylenepentanedioic acid,* or a mixture of two or three of the compounds can be obtained by dehydration of *3-hydroxy-3-methylpentanedioic acid.* Different reaction conditions such as temperature, solvent, concentration, and the nature of catalytic acids and/or bases, influence the dehydration mechanism and can be used to change the product mixture composition.

### Example 8 - Synthesis of 3-hydroxy-3-methylpentanedioic acid (MVLdiacid)

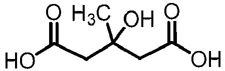

This compound is represented by Formula (IIb), (XIVc), (XVm), (XVn), (XVIm), (XVn), (XXb), and (XXVe). Synthesized by adaption of prior art: "Synthesis of Mevalonolactone from 4-(Hydroxyethyl)-4-methyl-1,3-dioxane." M. S. Sargsyan, A. T. Manukyan, S. A. Mkrtumyan, A. A. Gevorkyan Chem. Nat. Comp. 1990, 26 (1), 24 - 25.

To 20 mL 69-70 % nitric acid were added several drops of biobased MVL upon stirring. Once the start of the reaction was observed by a considerable darkening of the solution, the residual biobased MVL (4.1 g total) was added slowly with cooling of the solution in an ice-bath. After the completed addition, the solution was stirred at 40 °C for 4 h. All volatiles were distilled off at reduced pressures and temperature below 50 °C. The yellow oil was recrystallized from hot n-butyl acetate 2.9 g of the title compound as an off-white powder (57 % yield). ¹H NMR (500 MHz, DMSO) δ 9.87 (s, 3H), 2.44 (d, *J* = 13.4 Hz, 2H), 2.37 (d, *J* = 13.4 Hz, 2H), 1.20 (s, 3H).

### Synthesis of unsaturated polyester resins

### Example 9 - Synthesis of ortho UPR resin containing Formula (Ib) moieties

This polymer is represented by Formula (Ib), (III), and (V). A reaction vessel equipped with a thermocouple, distillation bridge, and nitrogen inlet was charged with 2.81 g phthalic anhydride (19 mmol), 2.78 g maleic anhydride (28 mmol), 0.90g 1,2-propylene glycol (12 mmol), 2.71 g 2-methylpropane-1,3-diol (30 mmol), and 0.93 g 3-methylpent-2-ene-1,5-diol (8 mmol). With slow nitrogen bubbling, the reaction was heated to 100 °C, the temperature was raised to 190 °C over the course of 2 hours, and let react at that temperature for 8 hours. The reaction was cooled to 130 °C, 2 mg 4-tert-butyl-catechol were added, and cooled to room temperature. The final resin had an acid number of 23 mg KOH/g, and Mₙ = 2.9 kg mol⁻¹, M_{w} / Mₙ = 10.92 (vs narrow PS standards in THF eluent).

### Example 10- Cross-linking of ortho UPR resin containing Formula (Ib) moieties

This thermoset is represented by Formula (Ib), (III), and (V). Styrene was added to the unsaturated polyester resin obtained in example 9 in a 40 : 60 weight by weight ratio, in addition to 4,N,N-trimethylaniline (0.08 weight%) and benzoyl peroxide (1.0 weight %). The formulation was mechanically stirred to achieve complete mixing, poured into aluminum dishes, and cured at 50 °C for 24 hours to obtain a hard plastic.

### Example 11 - Synthesis of ortho UPR resin containing Formula (IIa) and (IIb) moieties

This polymer is represented by Formula (IIa), (IIb), (III), and (V). A reaction vessel equipped with a thermocouple, distillation bridge, and nitrogen inlet was charged with 0.90 g 1,2-propylene glycol, 3.44 g 2-methylpropane-1,3-diol, 1.41 g phthalic anhydride, and 2.81 g maleic anhydride and heated under a stream of dry nitrogen to 130 °C for 1 hour. 1.55 g 3-hydroxy-3-methylpentanedioic acid and 1 mg monobutyltin oxide were added and the temperature increased to 190 °C over the course of 6 hours, and stirred at 190 °C for another 2 hours. After the desired acid number of was reached, the mixture was cooled to 100 °C, 1 mg 4-tert-butyl-catechol was added, and the resin discharged. The final resin had an acid number of 31 mg KOH/g, Mₙ = 1.9 kg mol⁻¹, and M_{w} / Mₙ = 3.07 (vs narrow PS standards in THF eluent). Less than 10 mol% of the 3-hydroxy-3-methylpentanedioic ester moieties were dehydrated in the final resin.

### Example 12 - Cross-linking of ortho UPR resin containing Formula (IIa) and (IIb) moieties

This thermoset is represented by Formula (IIa), (IIb), (III), and (V). Styrene (stabilized with radical inhibitor) was added to the unsaturated polyester resin obtained in example 11 in a 40 : 60 weight by weight ratio, in addition to 4,N,N-trimethylaniline (0.08 weight%) and benzoyl peroxide (1.0 weight %). The formulation was mechanically stirred to achieve complete mixing, poured into aluminum dishes, and cured at 50 °C for 24 hours to obtain a hard plastic.

### Example 13 - Synthesis of iso UPR resin containing Formula (Ib) moieties (Prophetic)

This polymer is represented by Formula (Ib), (IV), and (V). A reaction vessel equipped with reflux condenser and nitrogen inlet was charged with isophthalic acid (1.0 equivalents), 1,2-propylene glycol (2.1 - x equivalents), and 3-methylpent-2-ene-1,5-diol (x equivalents, x = 1.0, 2.1), and heated to 210 °C for the desired reaction time. The reaction mixture was allowed to cool to 170 °C, when maleic anhydride (1.2 equivalents) was added reaction is continued for another desired time, and then cooled to room temperature after addition of radical inhibitor.

### Example 14 - Cross-linking of iso UPR resin containing Formula (Ib) moieties (Prophetic)

This thermoset is represented by Formula (Ib), (IV), and (V). Styrene (stabilized with radical inhibitor) was added to the unsaturated polyester resin obtained in example 13 in a 40 : 60 weight by weight ratio, in addition to 4,N,N-trimethylaniline (0.08 weight%) and benzoyl peroxide (1.0 weight %). The formulation was mechanically stirred to achieve complete mixing, poured into Teflon molds, and cured under ambient conditions for 12 h, and/or at elevated temperatures of 100 °C for 6 h.

### Example 15- Synthesis of iso UPR resin containing Formula (IIa) moieties (Prophetic)

This polymer is represented by Formula (IIa), (IV), and (V). A reaction vessel equipped with reflux condenser and nitrogen inlet was charged with isophthalic acid (1.0 equivalents), 1,2-propylene glycol (2.1 equivalents), and 3-methylpent-2-enedioic acid (x equivalents, x = 0.6, 1.2), and heated to 210 °C for the desired reaction time. The reaction mixture was allowed to cool to 170 °C, when maleic anhydride (1.2 - x equivalents) was added reaction is continued for another desired time, and then cooled to room temperature after addition of radical inhibitor.

### Example 16 - Cross-linking of ortho UPR resin containing Formula (IIa) moieties (Prophetic)

This thermoset is represented by Formula (IIa), (IV), and (V). Styrene (stabilized with radical inhibitor) was added to the unsaturated polyester resin obtained in example 15 in a 40 : 60 weight by weight ratio, in addition to 4,N,N-trimethylaniline (0.08 weight%) and benzoyl peroxide (1.0 weight %). The formulation was mechanically stirred to achieve complete mixing, poured into Teflon molds, and cured under ambient conditions for 12 h, and/or at elevated temperatures of 100 °C for 6 h.

### Synthesis of polyurethane products

### Example 17 - Synthesis of polyurethane containing Formula (Id) with tin catalyst

This polymer is represented by Formula (Id), and (XIIIe). To 5 mL of a dimethylacetamide solution containing 0.250 g 3,5-dihydroxy-N-(2-hydroxyethyl)- 3-methylpentanamide and 0.327 g 4,4'-methylene diphenyl diisocyanate was added 1 mol% dibutyltin dilaureate catalyst, and the mixture was stirred under a blanket of nitrogen at 80 °C for 5 hours. The reaction was quenched by precipitation into an aqueous methanol solution (1:1 volume by volume ratio). The solid product was collected by filtration, washed with 50 mL methanol, and dried under vacuum at 70 °C for 24 h. (Yield = 90 %; GPC (DMF, 70 °C, vs narrow PS standards): Mₙ = 963 g mol⁻¹, M_{w} / Mₙ = 3.96; T_{g} = 92 °C).

### Example 18 - Synthesis of polyurethane containing Formula (Id) with acid catalyst

This polymer is represented by Formula (Id), and (XIIIe). To 1 mL of a dimethylacetamide solution containing 98 mg 3,5-dihydroxy-N-(2-hydroxyethyl)- 3-methylpentanamide and 129 mg 4,4'-methylene diphenyl diisocyanate was added 1 mol% para toluenesulfonic acid catalyst, and the mixture was stirred under a blanket of nitrogen for 24 hours. The reaction was quenched by precipitation into methanol. The solid product was collected by filtration, washed with 40 mL methanol, and dried under vacuum at 70 °C for 24 h. (Yield = 82 %; GPC (DMF, 70 °C, vs narrow PS standards): Mₙ = 11,224 g mol⁻¹, M_{w} / Mₙ = 3.70; T_{g} = 122 °C).

### Example 19 - Synthesis of polyurethane #2 containing Formula (Id) with tin catalyst

This polymer is represented by Formula (Id), and (XIIIa). To 1 mL of a dimethylacetamide solution containing 104 mg 3,5-dihydroxy-N-(2-hydroxyethyl)- 3-methylpentanamide and 83 µL hexamethylene diisocyanate was added 1 mol% dibutyltin dilaureate catalyst, and the mixture was stirred under a blanket of nitrogen for 6 hours. The reaction was quenched by precipitation into methanol. The waxy product was collected by centrifugation, washed with 40 mL methanol, and dried under vacuum at 70 °C for 24 h. (Yield = 80 %; GPC (DMF, 70 °C, vs narrow PS standards): Mₙ = 1,370 g mol⁻¹, M_{w} / Mₙ = 4.38; T_{g} = - 7 °C).

### Example 20 - Formulation of vinyl urethane (VU) resin

This thermoset is represented by Formula (Id), and (XIIIe). A mixture of 0.2 g (2 equivalents) 3,5-dihydroxy-N-(2-hydroxyethyl)-3-methylpentanamide (MVLea), 0.13 g (2 equivalents) (2-hydroxyethyl)methacrylate, 0.39 g (3 equivalents) 4,4'-methylene diphenyl diisocyanate, 0.5 mL styrene, 0.5 mg hydroquinone, and 0.5 uL dibutyltin dilaureate were stirred at 60 °C for 4 hours. After cooling to room temperature, the clear resin was promoted by addition of 4,N,N-trimethylaniline (0.08 weight%), and cured with benzoyl peroxide (1.0 weight %) at room temperature for 24 hours, to obtain a hard, clear solid.

### Example 21 - Formulation of vinyl urethane (VU) resin

This thermoset is represented by Formula (Id), and (XIIIe). A mixture of 0.2 g (2 equivalents) 3,5-dihydroxy-N-(2-hydroxyethyl)-3-methylpentanamide (MVLea), 0.13 g (2 equivalents) (2-hydroxyethyl)methacrylate, 0.39 g (3 equivalents) 4,4'-methylene diphenyl diisocyanate, 0.5 mL methyl acrylate, 0.5 mg hydroquinone, and 0.5 uL dibutyltin dilaureate were stirred at 60 °C for 4 hours. After cooling to room temperature, the clear resin was promoted by addition of 4,N,N-trimethylaniline (0.08 weight%), and cured with benzoyl peroxide (1.0 weight %) at room temperature for 24 hours, to obtain a hard, clear solid.

### Synthesis of pendent MVL polyacrylate

### Example 22 - Synthesis of polyacrylate containing Formula (VIII)

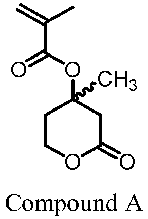

Compound A (90 mg) and azobisisobutyronitrile (3 mol%) were dissolved in 0.5 mL deoxygenated toluene and heated under a blanket of nitrogen for 3 h at 70 °C. The reaction was quenched by cooling to room temperature and opening the reaction vessel to ambient air. The polymer suspension was precipitated from methanol, filtered, washed with 15 mL methanol, and dried in vacuum at 70 °C for 24 h. (Yield: 85 %; GPC (DMF, 70 °C, vs narrow PS standards): Mₙ = 17,372 g mol⁻¹, M_{w} / Mₙ = 4.03).

### Example 23 - Synthesis of polyacrylate copolymers containing Formula (VIII) (prophetic)

The procedure of example 12 was applied to a system comprised of an equimolar mixture of compound A and (meth-)acrylic acid, (meth-)acrylamide, or methyl (meth-)acrylate.

### Example 24 - Synthesis of polyacrylate containing Formula (X) (prophetic)

A solution of a polymer from example 20 or 21 in a small volume of suitable solvent, i.e. dimethylformamide, is cooled to 0 °C, and mixed with a diamine (1 mol%, 5 mol%, 25 mol%, and/or 50 mol% per unit of compound A), i.e. hexamethylene diamine, or lysine. After the allotted amount of time, unreacted reagents are removed by washing with the employed solvent, followed by washing with a low boiling solvent, i.e. methanol, before drying of the cross-linked polymer in vacuum.

### Polyester/Polyesterol syntheses

### Example 25 - Synthesis of polyester from MVL-derived diol (prophetic)

This polymer is represented by Formula (Ib). A three-neck round bottom flask equipped with a vacuum adapter, nitrogen inlet and thermocouple was filled with 3.7 g (25 mmol) dimethyl succinate, 1.2 g (13 mmol) 1,4-butanediol, and 1.5 g (13 mmol) 3-methylpent-2-ene-1,5-diol. The mixture was sparged with dry nitrogen for 0.5 h, and heated to 130 °C. After 30 minutes, 0.1 mol% titanium(IV) tetraisopropoxide was added, and the temperature slowly raised to 180 °C under a continuous nitrogen stream. Vacuum (0.1 mbar) was gradually applied after another 3 hours, and the temperature was raised to 210 °C for 2 h. The cooled mixture was dissolved in 40 mL chloroform and precipitated from 250 mL vigorously stirred methanol. The oily residue was purified by decanting off the methanol phase, and 3 x 200 mL methanol washes, after which is was dried at elevated temperatures in vacuo to constant weight. Yield: 1.2 g. GPC: Mₙ = 5,655 g mol⁻¹, M_{w} / Mₙ = 3.22 vs. narrow PS standards in THF eluent.

### Example 26 - Synthesis of polyester from MVL-derived diacid (prophetic)

This polymer is represented by Formula (IIb). A three-neck round bottom flask equipped with distillation condenser, nitrogen inlet and thermocouple was filled with 6.0 g (37 mmol) 3-hydroxy-3-methylpentanedioic acid and 3.4 g (38 mmol) butane-1,4-diol, sparged with dry nitrogen, and heated to 130 °C. After 30 minutes, 0.1 mol% titanium(IV) tetraisopropoxide was added, and the temperature slowly raised to 180 °C under a continuous nitrogen stream. Vacuum (0.1 mbar) was gradually applied after another 3 hours, and the temperature was raised to 210 °C for 2 h. The cooled mixture was dissolved in 40 mL chloroform and precipitated from 250 mL vigorously stirred methanol. The white precipitate was filtered, washed with 3 x 200 mL methanol, and dried at elevated temperatures in vacuo overnight.

### Example 27 - Synthesis of polyester from MVLea

This polymer is represented by Formula (Id). To a solution of 0.5 g (2.6 mmol) MVLea in 3 mL dry dimethylacetamide in an oven dried one neck flask was added 0.76 mL dry triethylamine (2.1 equivalents, 5.5 mmol), and the reaction was cooled to 0 °C. Adipoyl chloride (0.37 mL, 0.9 equivalents) was added dropwise via syringe, resulting in the formation of precipitate. The reaction mixture was stirred for 4 h at that temperature after which 5 mL of 0.5 M HCl was added to quench the reaction, followed by 5 mL of dichloro methane. The phases were separated, and the organic phase further washed with 5 mL saturated ammonium chloride solution, and 5 mL brine. The organic phase was dried with magnesium sulfate, filtered, and evaporated to yield 0.65 g of polyester as a yellow oil. (GPC: Mₙ = 1,852 g mol⁻¹, M_{w} / Mₙ = 1.69 vs. narrow PS standards in THF)

### Example 28 - Synthesis of

### 2,2'-(((3-methylpent-2-ene-1,5-diyl)bis(oxy))bis(methylene))bis(oxirane) (prophetic)

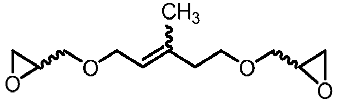

This compound is represented by Formula (XVc). In a round bottom flask equipped with reflux condenser was added 3.0 g (26 mmol) biobased 3-methylpent-2-ene-1,5-diol, 20 mL dry dimethyl sulfoxide, and 6.2 g (110 mmol) KOH. To this mixture was slowly added 12.2 mL (156 mmol) epichlorohydrin and the reaction was stirred at 50 °C for 16 h. After cooling back to room temperature the mixture was filtered, and the solvent removed under reduced pressure. The residue was partitioned between saturated sodium chloride solution and diethyl ether. The product was purified by vacuum distillation.

(Adapted from: Crivello, J. V. "Design and synthesis of multifunctional glycidyl ethers that undergo frontal polymerization." J. Polym. Sci. A Polym. Chem. 2006, 44, 6435 - 6448.)

### Example 29 - Synthesis of oxiran-2-ylmethyl 3-methyl-5-(oxiran-2-ylmethoxy)pent-2-enoate (prophetic)

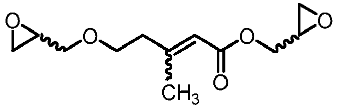

This compound is represented by Formula (XVk). In a round bottom flask equipped with reflux condenser was added 3.0 g (45 mmol) biobased AML, 40 mL dry dimethyl sulfoxide, and 9.3 g (165 mmol) KOH. To this mixture was slowly added 23.5 mL (0.30 mol) epichlorohydrin and the reaction was stirred at 50 °C for 16 h. After cooling back to room temperature the mixture was filtered, and the solvent removed under reduced pressure. The residue was partitioned between saturated sodium chloride solution and diethyl ether. The product was purified by vacuum distillation.

### Example 30 - 3-methyl-1,5-bis(vinyloxy)pent-2-ene (Prophetic)

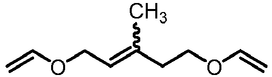

This compound is represented by Formula (XVIb). Into a reactor was charged 0.2 mol of biobased 3-methylpent-2-ene-1,5-diol, NaOH (7 mol%), and CsF (7 mol%), and acetylene was supplied to the initial pressure of 10 - 14 atm. The reaction was carried out at stirring and heating to 135 - 140°C for 15 h. After venting, the reaction product was purified by distillation at reduced pressure.

Alternative methods for a vinyl ether synthesis can be found in Winternheimer, D. J.; Shade, R. E.; Merlic, C. A. "Methods for Vinyl Ether Synthesis" Synthesis 2010, 15, 2497 - 2511.

### Example 31 - Synthesis of polycarbonate (Prophetic)

This polymer is represented by Formula (XVIIIb). A stirred reactor was charged with biobased 3-methylpent-2-ene-1,5-diol (1 mol), and diphenylcarbonate (1 mol) and initially held at 200°C at 20 mbar. The temperature is slowly raised to 290 - 310°C, and the pressure lowered to 0.1 mbar until the desired molecular weight is obtained.

### Example 32 - Synthesis of 6-methyl-3,7-dioxabicyclo[4.1.0]heptan-2-one (Prophetic)

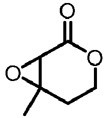

This compound is represented by Formula (XXVI). To a solution of biobased AML (6.7 g) in methanol (50 mL) at 0 °C was added 30% aqueous H₂O₂ (18.3 mL) over a 10 minute period. This was followed by the dropwise addition of 5N NaOH (0.4 mL). After stirring at ∼10 °C until full conversion was reached, the reaction was quenched with brine, extracted with dichloromethane and concentrated in vacuo. The crude product was purified by Kugelrohr distillation to yield the title compound as a clear, colorless oil. (Adopted from: Org. Lett., 2012, 14 (5), pp 1322-1325.)

It should be apparent to a person skilled in the art that the above compound represents a versatile monomer for ring-opening polymerization with concurrent or sub sequential crosslinking ability for the production of thermoset materials, amongst others.

### Example 33 - Ring-opening polymerization of Compound B (Prophetic)

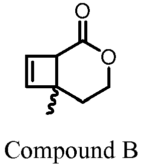

Compound B has been reported as the product of a photochemical [2+2] cycloaddition of acetylene with AML *(*J. Am. Chem. Soc. 1982, 104, 5486-5489).

To a stirred, dry solution of biobased compound B (1.0 equivalent) and benzyl alcohol (0.01 equivalent) was added triazabicyclodecene (0.005 equivalent) and stirred in a dry, inert atmosphere at room temperature, until a target molecular weight was reached. The reaction is then quenched by the addition of benzoic acid (0.005 eqvivalent), dissolved in chloroform, precipitated from methanol, washed with methanol, and dried in vacuo. The resulting polymer is represented by Formula (XXVII).

It should be apparent to a person skilled in the art that the above compound represents a versatile monomer for ring-opening polymerization with concurrent or sub sequential crosslinking ability for the production of thermoset materials such as unsaturated polyester resins, amongst others.

### Example 34 - Mevalonolactone production

Genetic segments encoding *mvaE* (acetyl-CoA acetyltransferase/HMG-CoA reductase, GenBank No. AAG02438) and *mvaS* (HMG-CoA synthase, GenBank No. AAG02439) in Enterococcus faecalis V583 were amplified from its genomic DNA (obtained from ATCC). These segments were inserted into a vector (with pBR322 origin back bone, Ampicillin marker, lacIq, rrnB transcription termination sequences) under the control of IPTG inducible Trc promoter-lac operator to obtain plasmid pSE1.

Chemically competent *E.coli* cells of XL-1Blue strain (endA1 gyrA96(nal^{R}) thi-1 recA1 relA1 lac glnV44 F'[::Tn10 proAB⁺ lacI^{q} Δ(lacZ)M15] hsdR17(r_{K}⁻ m_{K}⁺)) were transformed with plasmid pSE1 using the procedures outlined in Sambrook-Maniatis (Green, M. R.; Sambrook, J. Ed. Molecular Cloning: A Laboratory Manual, Fourth Edition, 2002) to obtain strain *E.coli-*SE1.

*E.coli*-SE1 strain was propagated in LB medium supplemented with 100µg/liter ampicillin in 250mL of media in a 1 liter conical flask incubated at 37°C in an orbital shaker at 220 rpm for 10 hours reaching an OD600 of 3. This was used as an inoculum for the production in an Infors 5lt bioreactor. 1.75 liter of production media (containing 15 g/l glucose, 7 g/l KH₂PO₄, 1 g/l NH₄Cl, 5 g/l yeast extract, 1 g/l citric acid, 10 mg MnSO₄, 2 g/l MgSO₄, 200 mg/l FeSO₄ and 10 mg/l thiamine·HCl) was combined with 250mL of inoculum in the bioreactor. The pH was maintained at 7 with 20% NH₄OH. Temperature was maintained at 32°C. Air was sparged at 2 liters per minute (LPM) and agitation was maintained at 700 rpm. 10 hours after inoculation, 1ml of 1M IPTG was added to the bioreactor. Anti-foam was added as needed. Glucose concentration was maintained around 10g/l, by addition of 600g/l glucose solution to the bioreactor at 2 hour intervals. The bioreactor run was stopped at 48 hours. Cells were separated from the broth by use of 0.45 micro filters to obtain clear broth. Mevalonolactone concentration was found to be 40g/l at end of the fermentation.

## Claims

1. A biobased polymer comprising recurring monomeric units synthesized from a polymer precursor compound of a biobased mevalonolactone-diol or -diacid or derivative thereof, said compound being selected from the group consisting of Formula (XXVa), Formula (XXVb), Formula (XXVc), Formula (XXVd), Formula (XXVe), Formula (XXVf), and combinations thereof: 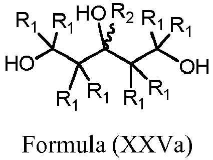 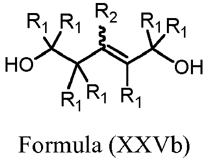 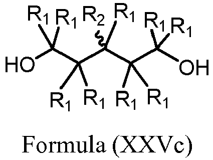 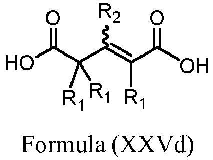 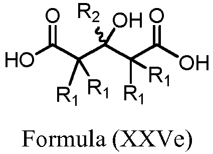 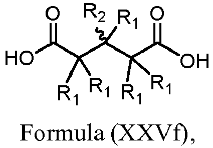 wherein, independently for each occurrence:
each R₁ is H or an alkyl; and
each R₂ is an alkyl.

2. The biobased polymer of claim 1, wherein each R₂ is selected from the group consisting of C₁-C₆ alkyls.

3. The biobased polymer of claim 1, wherein said compound has a 14C/12C ratio greater than zero measured as indicated in the description.

4. The biobased polymer of claim 1, wherein the compound is selected from the group consisting of Formula (XXVb), Formula (XXVc), Formula (XXVd), Formula (XXVe), Formula (XXVf), and combinations thereof.

5. The biobased polymer of claim 1, wherein the compound is selected from Formula (XXVc).

## Patentansprüche

1. Biobasiertes Polymer, umfassend wiederkehrende monomere Einheiten, das aus einer Polymervorläuferverbindung eines/einer biobasierten Mevalonolakton-Diols oder -Disäure oder einem Derivat davon synthetisiert ist, wobei die Verbindung aus der Gruppe ausgewählt ist, die aus Formel (XXVa), Formel (XXVb), Formel (XXVc), Formel (XXVd), Formel (XXVe), Formel (XXVf) und Kombinationen davon besteht: 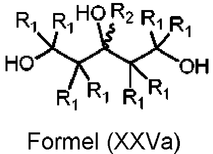 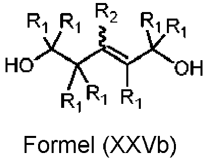 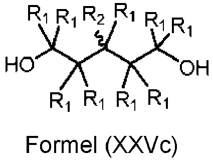 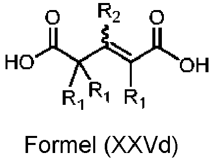 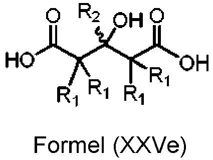 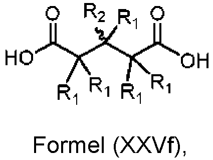 wobei unabhängig für jedes Vorkommen:
jedes R₁ H oder ein Alkyl ist; und
jedes R₂ ein Alkyl ist.

2. Biobasiertes Polymer nach Anspruch 1, wobei jedes R₂ aus der Gruppe ausgewählt ist, die aus C₁-C₆-Alkylen besteht.

3. Biobasiertes Polymer nach Anspruch 1, wobei die Verbindung ein 14C/12C-Verhältnis größer als null aufweist, das wie in der Beschreibung angegeben gemessen wird.

4. Biobasiertes Polymer nach Anspruch 1, wobei die Verbindung aus der Gruppe ausgewählt ist, die aus Formel (XXVb), Formel (XXVc), Formel (XXVd), Formel (XXVe), Formel (XXVf) und Kombinationen davon besteht.

5. Biobasiertes Polymer nach Anspruch 1, wobei die Verbindung aus Formel (XXVc) ausgewählt ist.

## Revendications

1. Polymère d'origine biologique comprenant des unités monomères récurrentes synthétisées à partir d'un composé précurseur polymère d'un mévalonolactone diol ou diacide ou dérivé de celui-ci d'origine biologique, ledit composé étant choisi dans le groupe constitué par la formule (XXVa), la formule (XXVb), la formule (XXVc), la formule (XXVd), la formule (XXVe), la formule (XXVf) et leurs combinaisons : 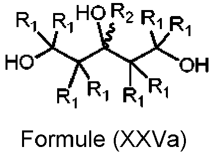 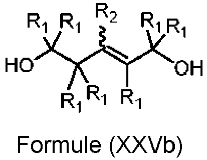 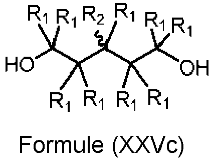 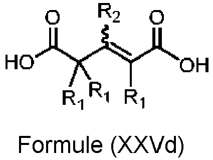 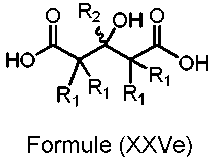 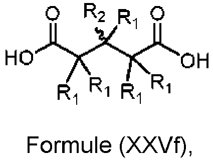 indépendamment pour chaque occurrence :
chaque R₁ représentant H ou un alkyle ; et
chaque R₂ représentant un alkyle.

2. Polymère d'origine biologique selon la revendication 1, dans lequel chaque R₂ est choisi dans le groupe constitué par les alkyles en C₁-C₆.

3. Polymère d'origine biologique selon la revendication 1, dans lequel ledit composé a un rapport 14C/12C supérieur à zéro mesuré comme indiqué dans la description.

4. Polymère d'origine biologique selon la revendication 1, dans lequel le composé est choisi dans le groupe constitué par la formule (XXVb), la formule (XXVc), la formule (XXVd), la formule (XXVe), la formule (XXVf) et leurs combinaisons.

5. Polymère d'origine biologique selon la revendication 1, dans lequel le composé est choisi parmi la formule (XXVc).
